(19) Europäisches Patentamt
European Patent Office
Office européen des brevets

(11) Veröffentlichungsnummer: **0 342 157 B1**

# (12) EUROPÄISCHE PATENTSCHRIFT

(45) Veröffentlichungstag der Patentschrift: **21.06.95**

(51) Int. Cl.[6]: **C07C 245/00**, C07C 249/00, C07C 303/00, C07C 307/00, C07C 309/00, C07C 327/00, C07C 331/00, G01N 33/68

(21) Anmeldenummer: **89810336.1**

(22) Anmeldetag: **01.05.89**

## (54) Aromatische Säuren.

(30) Priorität: **10.05.88 CH 1766/88**
**22.03.89 CH 1065/89**

(43) Veröffentlichungstag der Anmeldung:
**15.11.89 Patentblatt 89/46**

(45) Bekanntmachung des Hinweises auf die Patenterteilung:
**21.06.95 Patentblatt 95/25**

(84) Benannte Vertragsstaaten:
**AT BE CH DE ES FR GB GR IT LI LU NL SE**

(56) Entgegenhaltungen:
**EP-A- 0 053 915**
**DE-A- 2 329 388**
**US-A- 3 657 220**
**US-A- 4 110 444**

**THE JOURNAL OF BIOLOGICAL CHEMISTRY, Band 264, Nr. 6, 25. Februar 1989, S. 3111-3115, US; J.-Y. CHANG: "Binding of heparin to human antithrombin III activates selective chemical modification at lysine 236"**

(73) Patentinhaber: **CIBA-GEIGY AG**
**Klybeckstrasse 141**
**CH-4002 Basel (CH)**

(72) Erfinder: **Chang, Jui Yoa, Dr.**
**Morgentalstrasse 5**
**CH-4416 Bubendorf (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft neue aromatische Säuren, insbesondere Verbindungen der Formel

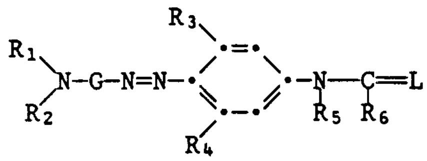 (I),

worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine gegebenenfalls substituierte 1,4-Phenylengruppe oder eine gegebenenfalls substituierte 1,4-Naphthylengruppe steht und worin entweder $R_5$ und $R_6$ gemeinsam für eine zusätzliche Bindung stehen und L ein Sauerstoff- oder ein Schwefelatom bedeutet oder worin $R_5$ Wasserstoff ist, $R_6$ Halogenmethyl bedeutet und L für ein Sauerstoffatom steht, und ihre Salze, die Verwendung der Verbindungen I und ihrer Salze, ein Verfahren zur Herstellung der Verbindungen I und ihrer Salze, in diesem Herstellungsverfahren verwendete Ausgangsstoffe und deren Salze, ein Verfahren zur Herstellung dieser Ausgangsstoffe und ihrer Salze, eine Vorrichtung, in welcher die Verbindungen I und ihre Salze Verwendung finden, und ein Verfahren, in welchem diese Vorrichtung Verwendung findet.

Als gegebenenfalls substituierte 1,4-Phenylengruppen kommen beispielsweise gegebenenfalls durch Carboxy und/oder Sulfo substituierte 1,4-Phenylengruppen in Betracht, wobei die substituierten 1,4-Phenylengruppen 1 bis und mit 4, insbesondere 1 oder 2, der genannten Substituenten besitzen können. Weisen die substituierten 1,4-Phenylengruppen mehr als einen Substituenten auf, so können mehrere oder alle dieser Substituenten gleich sein. Beispielhaft genannt seien die 2-Sulfo-, 2,3-, 2,5- und 3,5-Disulfo-, 2,3,5-Trisulfo- und 2,3,5,6-Tetrasulfo-1,4-phenylengruppe, die 2-Carboxy-, 2,3-, 2,5- und 3,5-Dicarboxy-, 2,3,5-Tricarboxy- und 2,3,5,6-Tetracarboxy-1,4-phenylengruppe, die 2-Carboxy-3-sulfo-, 2-Carboxy-5-sulfo-, 3-Carboxy-5-sulfo-, 2,3-Dicarboxy-5-sulfo-, 3,5-Dicarboxy-2-sulfo-, 5-Carboxy-2,3-disulfo- und 2-Carboxy-3,5-disulfo-1,4-phenylengruppe und insbesondere die 1,4-Phenylengruppe.

Als gegebenenfalls substituierte 1,4-Naphthylengruppen kommen beispielsweise gegebenenfalls durch Carboxy und/oder Sulfo substituierte 1,4-Naphthylengruppen in Betracht, wobei die substituierten 1,4-Naphthylengruppen 1 bis und mit 6, insbesondere 1 bis und mit 3, der genannten Substituenten besitzen können. Weisen die substituierten 1,4-Naphthylengruppen mehr als einen Substituenten auf, so können mehrere oder alle dieser Substituenten gleich sein. Beispielhaft genannt seien die 2-, 5- und 6-Sulfo-, 2,3-, 5,6-, 6,7- und 2,6-Disulfo-, 2,3,5- und 2,3,6-Trisulfo- und 2,3,5,7- und 2,3,6,7-Tetrasulfo-1,4-naphthylengruppe, die 2-, 5- und 6-Carboxy-, 2,3-, 5,6-, 6,7- und 2,6-Dicarboxy-, 2,3,5- und 2,3,6-Tricarboxy- und 2,3,5,7- und 2,3,6,7-Tetracarboxy-1,4-naphthylengruppe, die 2-Carboxy-3-sulfo-, 2-Carboxy-5-sulfo-, 3-Carboxy-6-sulfo-,5-Carboxy-7-sulfo-, 2,3-Dicarboxy-5-sulfo-, 3,5-Dicarboxy-2-sulfo-, 6,7-Dicarboxy-2-sulfo-, 3-Carboxy-6,7-disulfo-, 5-Carboxy-2,3-disulfo- und 2-Carboxy-3,5-disulfo-1,4-naphthylengruppe und insbesondere die 1,4-Naphthylengruppe.

Die Erfindung betrifft beispielsweise Verbindungen I, worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine gegebenenfalls substituierte 1,4-Phenylengruppe oder eine gegebenenfalls substituierte 1,4-Naphthylengruppe steht, $R_5$ und $R_6$ gemeinsam für eine zusätzliche Bindung stehen und L ein Sauerstoff- oder ein Schwefelatom bedeutet, und ihre Salze.

Die Verbindungen I können teilweise in Form von Stereoisomeren vorliegen. Wenn die Verbindungen I beispielsweise mindestens ein chirales Kohlenstoffatom (C-Atom) besitzen (z.B. ein C-Atom eines entsprechenden Restes $R_1$), können sie z.B. als reine Enantiomere oder Enantiomerengemische, wie Racemate, und, sofern noch mindestens ein weitere chirales Zentrum vorhanden ist (z.B. ein C-Atom eines entsprechenden Restes $R_2$), auch als Diastereomere, Diastereomerengemische oder Racematgemische vorliegen.

Salze von Verbindungen I sind insbesondere Salze mit Basen, vorzugsweise pharmazeutisch verwendbare Salze mit Basen, beispielsweise Alkalimetall- oder Erdalkalimetallsalze, z.B. Natrium-, Kalium- oder Magnesiumsalze, Uebergangsmetallsalze, wie Zink- oder Kupfersalze, oder Salze mit Ammoniak oder organischen Aminen, wie cyclischen Aminen, wie Mono-, Di- bzw. Triniederalkylaminen, wie Hydroxyniederalkylaminen, z.B. Mono-, Di- bzw. Trihydroxyniederalkylaminen, Hydroxyniederalkyl-niederalkylaminen oder Polyhydroxyniederalkylaminen. Cyclische Amine sind z.B. Morpholin, Thiomorpholin, Piperidin oder Pyrrolidin. Als Mononiederalkylamine kommen beispielsweise Aethyl- oder t-Butylamin, als Diniederalkylamine beispielsweise Diäthyl- oder Diisopropylamin, als Triniederalkylamine beispielsweise Trimethyl- oder Triäthylamin in Betracht. Entsprechende Hydroxyniederalkylamine sind z.B. Mono-, Di- oder Tri-äthanolamin und Hydroxyniederalkyl-niederalkyl-amine sind z.B. N,N-Dimethylamino- oder N,N-Diäthylamino-äthanol, während als Polyhydroxyniederalkylamin z.B. Glucosamin in Frage kommt. Die Verbindungen I können ferner Säureadditionssalze, vorzugsweise pharmazeutisch verwendbare Säureadditionssalze, beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, bilden. Die Verbindungen I können auch innere Salze bilden.

Umfasst sind ferner für pharmazeutische Verwendungen weniger geeignete Salze von Verbindungen I. Diese können beispielsweise für die Isolierung oder Reinigung von freien erfindungsgemässen Verbindungen I sowie von deren pharmazeutisch verwendbaren Salzen verwendet werden.

Vor- und nachstehend sind unter mit "Nieder" bezeichneten Resten oder Verbindungen, sofern nicht abweichend definiert, insbesondere solche zu verstehen, die bis und mit 7, vor allem bis und mit 4, Kohlenstoffatome enthalten.

Niederalkyl ist z.B. $C_1$-$C_4$-Alkyl, wie Methyl, Aethyl, n-Propyl, Isopropyl, n-Butyl, Isobutyl, sek-Butyl oder tert-Butyl, und umfasst ferner $C_5$-$C_7$-Alkyl-, d.h. Pentyl-, Hexyl- oder Heptylreste.

Halogenmethyl ist Fluor-, Chlor- oder Brommethyl, insbesondere aber Iodmethyl.

Die Verbindungen I und ihre Salze weisen wertvolle Eigenschaften auf. Sie lassen sich beispielsweise als Hilfsmittel bei der Untersuchung von Proteinen einsetzen, z.B. als Reagentien für die chemische Modifizierung von Proteinen. Der Begriff "Protein" ist dabei vorstehend und nachfolgend so zu verstehen, dass er sowohl Peptide mit einer relativen Molekularmasse von 10000 oder mehr Atommasseneinheiten, die allgemein als Proteine bezeichnet werden, als auch Peptide mit einer relativen Molekularmasse von weniger als 10000 Atommasseneinheiten, die allgemein als Polypeptide bezeichnet werden, umfasst, wobei für die untere Grenze der relativen Molekularmasse der Polypeptide ein Wert von etwa 1000 bis etwa 2000 Atommasseneinheiten gelten soll. Beispielhaft seien aus der Vielzahl der Anwendungen, bei denen die chemische Modifizierung von Proteinen eine Rolle spielen kann, die Strukturbestimmung und die Anfärbung von Proteinen herausgegriffen.

Bekanntermassen kann schon die Modifizierung eines einzigen oder einiger weniger seiner Aminosäurebausteine die räumliche Struktur eines Proteins und damit auch seine Funktion, z.B. seine biologische Aktivität, wesentlich verändern. Damit eröffnet sich die Möglichkeit, die gezielte chemische Modifizierung von Aminosäurebausteinen als weithin anwendbares Verfahren zur Bestimmung des Beitrages, den diese Bausteine zur räumlichen Struktur eines Proteins leisten, einzusetzen. Hierbei stellt sich das Problem, die aufgrund einer bestimmten chemischen Modifizierung von Aminosäurebausteinen gegebenenfalls eintretende Aenderung der räumlichen Proteinstruktur mit Art und Ausmass dieser chemischen Modifizierung in Beziehung zu setzen. Dazu muss die Struktur eines entsprechenden chemisch modifizierten Proteins bestimmt und mit der vor Durchführung der chemischen Modifizierung vorliegenden Struktur verglichen werden. Eines der Ziele einer solchen Strukturbestimmung ist es, festzustellen, in welcher Weise sich die Protein-Primärstruktur bei der chemischen Modifizierung verändert hat, d.h. welche Aminosäurebausteine chemisch modifiziert worden sind. Vielfach geht man bei derartigen Bestimmungen der Primärstruktur in der Weise vor, dass man ein chemisch modifiziertes Protein $M_a$, das nach der chemischen Modifizierung eines entsprechenden nicht-modifizierten Proteins $N_a$ erhalten wird, und das nicht-modifizierte Protein $N_a$ jeweils, gegebenenfalls nach Durchführung eines eventuell nötigen Denaturierungsschrittes, mit derselben Protease behandelt, die beiden so erhaltenen Peptidgemische $M_a^1$ und $N_a^1$ jeweils der hochauflösenden Flüssigkeitschromatographie (high-performance liquid chromatography; HPLC) unterwirft und die Peak-Muster in den beiden resultierenden Chromatogrammen $M_a^2$ und $N_a^2$ miteinander vergleicht. Dabei finden für die Aufnahme dieser Chromatogramme üblicherweise Detektionssysteme Verwendung, die das Lichtabsorptionsverhalten der Peptide auswerten. Das Peak-Muster im Chromatogramm $M_a^2$ unterscheidet sich nun in der Regel an den Stellen, an denen Peptide $M_a^3$ detektiert werden, die mindestens einen chemisch modifizierten Aminosäurebaustein enthalten, von dem Peak-Muster im Chromatogramm $N_a^2$ , da die Peptide $M_a^3$ im Regelfall ein anderes Lichtabsorptionsverhalten als die entsprechenden nicht-modifizierten Peptide $N_a^3$ zeigen. Die Peptide $M_a^3$ werden abgetrennt und der weiteren Primärstruktur-Analyse, z.B. der Aminosäure-Sequenzanalyse, unterworfen. Auf diese Weise kann im Idealfall jeder chemisch modifizierte Aminosäurebaustein identifiziert und charakterisiert werden.

Die Brauchbarkeit des vorstehend beschriebenen Verfahrens lässt jedoch in vielen Fällen zu wünschen übrig. So sind z.B. bei höherer relativer Molekularmasse des zu untersuchenden Proteins im Normalfall nach der Proteasebehandlung im entsprechenden Peptidgemisch so viele verschiedene Peptide enthalten,

dass bei der HPLC keine ausreichende Auftrennung des Peptidgemisches mehr erreichbar ist. Das weiteren ist die chemische Modifizierung selbst oftmals zu komplex; sie läuft vielfach unspezifisch ab, betrifft also sehr viele strukturell verschiedene oder gar alle Aminosäurebausteine des zu untersuchenden Proteins; sie sollte jedoch mit Vorteil möglichst spezifisch durchgeführt werden können, also gezielt nur auf eine möglichst genau umschriebene, vorteilhafterweise bezüglich Art und Anzahl eng definierte, Klasse von Aminosäurebausteinen gerichtet sein.

Es ist daher wünschenswert und von grossem praktischem Interesse, das vorstehend beschriebene Verfahren durch Ueberwindung der aufgeführten Nachteile zu optimieren. Die im Rahmen der vorliegenden Erfindung offenbarten Verbindungen I und ihre Salze machen eine solche Optimierung möglich. Diese Optimierung wird im einzelnen nachfolgend anhand der Verbindungen I, in welchen $R_1$, $R_2$, $R_3$, $R_4$ und G die vorstehend erwähnten Bedeutungen haben, $R_5$ und $R_6$ gemeinsam für eine zusätzliche Bindung stehen und L ein Sauerstoff- oder ein Schwefelatom bedeutet, und ihrer Salze erläutert. Diese Verbindungen werden nachstehend als Verbindungen IA bezeichnet.

Zunächst reagieren die Verbindungen IA und ihre Salze bei der chemischen Modifizierung eines Proteins, d.h. bei der Umsetzung mit diesem Protein, praktisch ausschliesslich mit den Aminogruppen in $\epsilon$-Stellung von Lysin-Bausteinen, wobei eine hohe Reaktivität zu beobachten ist. Nur in Einzelfällen erfolgt zusätzlich eine Reaktion mit der Aminogruppe des N-terminalen Aminosäurebausteins des betreffenden Proteins. Die von einer chemischen Modifizierung durch eine Verbindung IA oder ein Salz davon betroffenen Aminosäurebausteine eines Proteins sind also nach Art und Anzahl eindeutig und in vorteilhafter Weise eng begrenzt. So ist die Zahl der chemisch modifizierten Aminosäurebausteine in der Regel nicht grösser als die Zahl der Lysin-Bausteine im zu untersuchenden Protein. Sie kann lediglich im Einzelfall bei zusätzlicher N-terminaler Modifizierung noch um 1 grösser sein.

Weiterhin hat die Verwendung einer Verbindung IA oder eines Salzes davon für die chemische Modifizierung eines Proteins den grossen Vorteil, dass nach der Proteasebehandlung des resultierenden chemisch modifizierten Proteins $M_b$ ein Peptidgemisch $M_b^1$ erhalten wird, welches mittels einfacher hochauflösender Umkehrphasen-Flüssigkeitschromatographie (reversed phase HPLC) einer, in den meisten Fällen vollständigen, Abtrennung der Peptide $M_b^3$ , die mindestens einen chemisch modifizierten Aminosäurebaustein enthalten, von den übrigen Peptiden $N_b^3$ zugänglich ist.

Zudem lassen sich die in dem Peptidgemisch $M_b^1$ enthaltenen Peptide $M_b^3$ , welche mindestens einen chemisch modifizierten Aminosäurebaustein enthalten, einerseits und die nicht-modifizierten Peptide $N_b^3$ andererseits jeweils bei deutlich verschiedenen Wellenlängen detektieren, da die Peptide $M_b^3$ ein Lichtabsorptionsverhalten zeigen, das deutlich verschieden ist von dem der Peptide $N_b^3$ . Dies erleichtert die Identifizierung der Peptide $M_b^3$ erheblich. So können die Peptide $M_b^3$ , da sie farbig sind, vorteilhaft bei Wellenlängen des sichtbaren Lichtes detektiert werden, d.h. bei Wellenlängen zwischen etwa 400 und etwa 800 nm, z.B. in sauer Lösung bei einer Wellenlänge von 535 nm oder in alkalischer Lösung bei einer Wellenlänge von 465 nm, vorzugsweise bei einer der in den Beispielen 10 bis 15 angegebenen Wellenlängen. Die farblosen Peptide $N_b^3$ lassen sich hingegen bei Wellenlängen des ultravioletten Lichtes, z.B. bei einer Wellenlänge von 200, 220 oder 280 nm, detektieren, stören also die Detektion der farbigen Peptide $M_b^3$ nicht.

Des weiteren ist von besonderem Interesse, dass nicht nur die Peptide $M_b^3$ farbig sind, sondern auch das entsprechende chemisch modifizierte Protein $M_b$, aus dem die Peptide $M_b^3$ durch Proteasebehandlung entstehen. Da die chemische Modifizierung eines Proteins durch eine Verbindung IA oder ein Salz davon also stets mit einer Anfärbung des Proteins verbunden ist, können die Verbindungen IA und ihre Salze auch als Reagentien für die Anfärbung von Proteinen verwendet werden. Die durch Reaktion der $\epsilon$-Aminogruppen von Lysin-Bausteinen sowie in einzelnen Fällen zusätzlich durch Reaktion der Aminogruppe des N-terminalen Aminosäurebausteins entsprechender, farbloser, nicht-modifizierter, Proteine $N_b$ mit einer Verbindung IA oder einem Salz davon erhältlichen angefärbten Proteine $M_b$ lassen sich in vielfacher Weise zu analytischen und/oder diagnostischen Zwecken gebrauchen. Hinsichtlich Abtrennung und Detektion der angefärbten Proteine $M_b$ gelten dabei die vorstehenden Ausführungen für Abtrennung und Detektion farbiger Peptide $M_b^3$ in analoger Weise. Auch die Peptide $M_b^3$ sind vielfach zu analytischen und/oder diagnostischen Zwecken verwendbar.

Da Untersuchungen von Proteinen im allgemeinen in wässriger oder Wasser enthaltender Lösung erfolgen, besitzen die Verbindungen IA und ihre Salze in ihrer guten Wasserlöslichkeit eine weitere wertvolle Eigenschaft. Weil sich auch die chemisch modifizierten Proteine $M_b$ und die Peptide $M_b^3$ , die mindestens einen chemisch modifizierten Aminosäurebaustein enthalten, durch gute Wasserlöslichkeit auszeichnen, können sowohl die Umsetzung von Proteinen $N_b$ mit einer Verbindung IA oder einem Salz davon als auch gegebenenfalls beabsichtigte weitere, bei Untersuchungen von Proteinen übliche, Verfahrensschritte, z.B. der nachfolgend erwähnten Art, mit Vorteil in wässriger Lösung, gegebenenfalls unter

Zusatz eines organischen Lösungsmittels, durchgeführt werden.

Im Zuge der Umsetzung mit einer Verbindung IA oder einem Salz davon werden die entsprechenden Aminogruppen eines Proteins $N_b$ der allgemeinen Formel $H_2N$-$R_x$ (Ia), worin $R_x$ jeweils für den Rest des Proteins steht, also die $\epsilon$-Aminogruppen von Lysin-Bausteinen sowie in einzelnen Fällen zusätzlich die Aminogruppen des N-terminalen Aminosäurebausteins, in Carbamyl- bzw. Thiocarbamylgruppen umgewandelt, so dass ein chemisch modifiziertes Protein der Formel

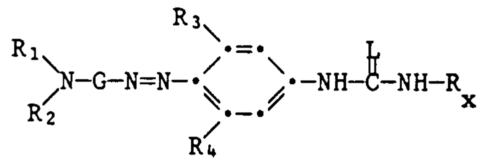

(Ib),

worin $R_1$, $R_2$, $R_3$, $R_4$, $R_x$ und G die vorstehend erwähnten Bedeutungen haben und L ein Sauerstoff- oder ein Schwefelatom darstellt, erhalten wird. Die Geschwindigkeit dieser Umwandlungsreaktion nimmt dabei mit höherer Temperatur und höherem pH-Wert des Reaktionsmediums zu. Typische Reaktionsbedingungen können beispielhaft aus den Beispielen 9 bis 15 entnommen werden. Die Stabilität der Carbamyl- bzw. Thiocarbamylbindungen in den Proteinen Ib ist vorteilhafterweise im Normalfall so gross, dass die Proteine Ib ohne wesentliche Zersetzung über Zeiträume von mehreren Tagen gelagert werden können und auch weitere, bei Untersuchungen von Proteinen übliche, Verfahrensschritte, wie chromatographische Trennungen, z.B. mittels molekularer Ausschluss-Chromatographie, beispielsweise Gelchromatographie, oder HPLC, wie enzymatische Behandlungen, z.B. die Behandlung mit einer Protease, beispielsweise mit Trypsin oder Chymotrypsin, wie andere Standardreaktionen, beispielsweise Denaturierungsschritte, z.B. die Reduktion von Disulfidbrücken und die anschliessende Carboxymethylierung der Mercaptogruppen, oder wie Testreaktionen zur Analyse der biologischen Aktivität, unversehrt überstehen. Solche Verfahrensschritte sind bekannt oder können in Analogie zu bekannten Verfahrensschritten durchgeführt werden. Beispielhafte Einzelheiten zu derartigen Verfahrensschritten gehen ebenfalls aus den Beispielen 9 bis 15 hervor. Hinsichtlich der Stabilität der Carbamyl- bzw. Thiocarbamylbindungen in den Peptiden $M_b^3$ gilt das vorstehend für die Proteine Ib Gesagte in analoger Weise.

Für die vorstehend beschriebene Optimierung des Verfahrens zur chemischen Modifizierung von Proteinen und zur Bestimmung der Primärstruktur von solchen chemisch modifizierten Proteinen können ausser den Verbindungen IA und deren Salzen auch Verbindungen I, worin $R_1$, $R_2$, $R_3$, $R_4$ und G die vorstehend erwähnten Bedeutungen haben, $R_5$ Wasserstoff ist, $R_6$ Halogenmethyl darstellt und L für ein Sauerstoffatom steht, und deren Salze verwendet werden. Nachstehend werden diese Verbindungen als Verbindungen IB bezeichnet.

Für die Verbindungen IB und deren Salze gilt im allgemeinen das vorstehend für die Verbindungenen IA und deren Salze Gesagte in analoger Weise. Allerdings reagieren die Verbindungen IB und deren Salze bei der chemischen Modifizierung eines Proteins, d.h. bei der Umsetzung mit diesem Protein, weder mit den $\epsilon$-Aminogruppen von Lysin-Bausteinen noch mit der Aminogruppe des N-terminalen Aminosäurebausteins dieses Proteins. Vielmehr werden im Zuge der Umsetzung mit einer Verbindung IB oder einem Salz davon spezifisch die Mercaptogruppen von Cystein-Bausteinen eines Proteins $N_c$ der allgemeinen Formel HS-$R_y$ (Id), worin $R_y$ jeweils für den Rest des Proteins steht, in Carbonylmethylthiogruppen umgewandelt, so dass ein chemisch modifiziertes Protein der Formel

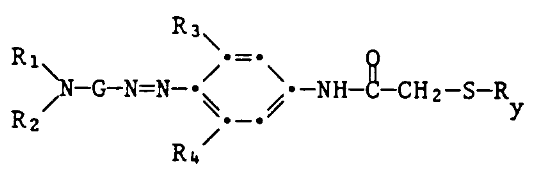

(Ie),

worin $R_1$, $R_2$, $R_3$, $R_4$, $R_y$ und G die vorstehend erwähnten Bedeutungen haben, erhalten wird. Somit sind bei Verwendung von Verbindungen IB und deren Salzen die von einer chemischen Modifizierung betroffenen Aminosäurebausteine in der Regel ebenfalls nach Art und Anzahl eindeutig und in vorteilhafter Weise eng begrenzt, wobei die Anzahl der chemisch modifizierten Aminosäurebausteine nicht grösser als die Zahl der Cystein-Bausteine im zu untersuchenden Protein ist.

Ein weiterer Gegenstand der Erfindung ist somit die Verwendung von Verbindungen I und ihren Salzen als Hilfsmittel bei der Untersuchung von Proteinen, z.B. als Reagentien für die chemische Modifizierung von Proteinen, insbesondere als Reagentien für die mit einer Anfärbung verbundene chemische Modifizierung von Proteinen. Dabei kann auch die gewerbsmässige Herrichtung der Hilfsmittel eingeschlossen sein.

Ebenfalls ein Gegenstand der vorliegenden Erfindung ist ein entsprechendes Verfahren für die chemische Modifizierung von Proteinen, insbesondere ein Verfahren für die mit einer Anfärbung verbundene chemische Modifizierung von Proteinen, das dadurch gekennzeichnet ist, dass man die Proteine mit einer Verbindung I oder einem Salz davon umsetzt.

Die Erfindung betrifft in erster Linie Verbindungen der Formel I, worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine unsubstituierte oder durch Carboxy und/oder Sulfo substituierte 1,4-Phenylengruppe oder eine unsubstituierte oder durch Carboxy und/oder Sulfo substituierte 1,4-Naphthylengruppe steht und worin entweder $R_5$ und $R_6$ gemeinsam für eine zusätzliche Bindung stehen und L ein Sauerstoff- oder ein Schwefelatom bedeutet oder worin $R_5$ Wasserstoff ist, $R_6$ Halogenmethyl bedeutet und L für ein Sauerstoffatom steht, und ihre Salze.

Die Erfindung betrifft insbesondere Verbindungen der Formel I, worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine unsubstituierte oder durch Carboxy und/oder Sulfo substituierte 1,4-Phenylengruppe oder eine unsubstituierte oder durch Carboxy und/oder Sulfo substituierte 1,4-Naphthylengruppe steht, $R_5$ und $R_6$ gemeinsam für eine zusätzliche Bindung stehen und L eine Sauerstoff- oder ein Schwefelatom bedeutet, und ihre Salze.

Die Erfindung betrifft ganz besonders Verbindungen der Formel I, worin $R_1$ $C_1$-$C_4$-Alkyl, wie Methyl oder Aethyl, darstellt, $R_2$ $C_1$-$C_4$-Alkyl, wie Methyl oder Aethyl, ist, $R_3$ Wasserstoff oder Sulfo bedeutet, $R_4$ Sulfo ist, G eine unsubstituierte oder durch Sulfo substituierte 1,4-Phenylengruppe darstellt und worin entweder $R_5$ und $R_6$ gemeinsam für eine zusätzliche Bindung stehen und L ein Sauerstoff- oder ein Schwefelatom bedeutet oder worin $R_5$ Wasserstoff ist, $R_6$ Iodmethyl bedeutet und L für ein Sauerstoffatom steht, und ihre Salze.

Die Erfindung betrifft vor allem Verbindungen der Formel I, worin $R_1$ $C_1$-$C_4$-Alkyl, wie Methyl oder Aethyl, darstellt, $R_2$ $C_1$-$C_4$-Alkyl, wie Methyl oder Aethyl, ist, $R_3$ Wasserstoff oder Sulfo bedeutet, $R_4$ Sulfo ist, G eine unsubstituierte oder durch Sulfo substituierte 1,4-Phenylengruppe darstellt, $R_5$ und $R_6$ gemeinsam für eine zusätzliche Bindung stehen und L ein Sauerstoff- oder ein Schwefelatom bedeutet, und ihre Salze.

Die Erfindung betrifft insbesondere Verbindungen der Formel I, worin $R_1$ $C_1$-$C_4$-Alkyl, wie Methyl, darstellt, $R_2$ $C_1$-$C_4$-Alkyl, wie Methyl, ist, $R_3$ Wasserstoff bedeutet, $R_4$ für Sulfo steht, G eine unsubstituierte 1,4-Phenylengruppe darstellt und worin entweder $R_5$ und $R_6$ gemeinsam für eine zusätzliche Bindung stehen und L ein Schwefelatom bedeutet oder worin $R_5$ Wasserstoff ist, $R_6$ Iodmethyl bedeutet und L für ein Sauerstoffatom steht, und ihrer Salze.

Die Erfindung betrifft in allererster Linie Verbindungen der Formel I, worin $R_1$ $C_1$-$C_4$-Alkyl, wie Methyl, darstellt, $R_2$ $C_1$-$C_4$-Alkyl, wie Methyl, ist, $R_3$ Wasserstoff bedeutet, $R_4$ für Sulfo steht, G eine unsubstituierte 1,4-Phenylengruppe darstellt, $R_5$ und $R_6$ gemeinsam für eine zusätzliche Bindung stehen und L ein Schwefelatom bedeutet, und ihre Salze.

Die Erfindung betrifft namentlich die in den Beispielen genannten neuen Verbindungen der Formel I und ihre Salze.

Ebenfalls Gegenstand der vorliegenden Erfindung ist ein Verfahren zur Herstellung einer Verbindung I oder eines Salzes davon, z.B. dadurch gekennzeichnet, dass man in einer Verbindung der Formel

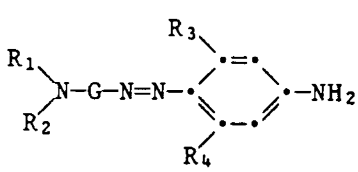

(II)

oder einem Salz davon die $NH_2$-Gruppe in die Gruppe der Formel

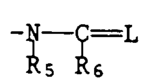

(Ic)

überführt und gewünschtenfalls ein verfahrensgemäss erhältliches Isomerengemisch in die Komponenten auftrennt und das gewünschte Isomere I isoliert, ein verfahrensgemäss erhältliches Enantiomeren- oder Diastereomerengemisch in die Enantiomeren oder Diastereomeren aufspaltet und das gewünschte Enantiomere oder Diastereomere isoliert und/oder eine verfahrensgemäss erhältliche freie Verbindung I in ein Salz überführt oder ein verfahrensgemäss erhältliches Salz in die freie Verbindung I oder in ein anderes Salz umwandelt.

Die vor- und nachstehend beschriebenen Umsetzungen werden in an sich bekannter Weise durchgeführt, z.B. in Gegenwart eines Katalysators und/oder in Ab- oder üblicherweise in Anwesenheit eines geeigneten inerten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80 °C bis zur Siedetemperatur des Reaktionsmediums, vorzugsweise von etwa -20 °C bis etwa +150 °C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln II, IIa, III, IV, IVa und V, das bei der Herstellung der Verbindungen I oder ihrer Salze Verwendung findet, ist zum Teil bekannt oder kann nach an sich bekannten Methoden hergestellt werden.

Ausgangsmaterial mit basischen Zentren kann z.B. in Form von Säureadditionssalzen, beispielsweise mit den vorstehend aufgeführten Säuren, vorliegen, während Ausgangsverbindungen mit sauren Gruppen Salze mit Basen, z.B. der vorstehend genannten Art, bilden können.

Die Ueberführung der $NH_2$-Gruppe in einer Verbindung II oder einem Salz davon in eine Gruppe Ic kann beispielsweise so durchgeführt werden, dass man die Verbindung II oder ein Salz davon mit einer Verbindung der Formel

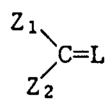

(IIa),

worin entweder L für ein Sauerstoff- oder ein Schwefelatom steht und $Z_1$ und $Z_2$ entweder unabhängig voneinander jeweils für eine nucleofuge Abgangsgruppe $G_1$ oder gemeinsam für gegebenenfalls funktionell abgewandeltes Oxo $G_2$ stehen oder worin L für ein Sauerstoffatom steht, $Z_1$ eine nucleofuge Abgangsgruppe $G_1$ ist und $Z_2$ Halogenmethyl bedeutet, umsetzt.

Nucleofuge Abgangsgruppen $G_1$ sind beispielsweise gegebenenfalls verätherte oder veresterte Hydroxy- oder Mercaptogruppen, ferner Amino-, Ammonium- oder Sulfoniumgruppen. Veräthertes Hydroxy ist beispielsweise Niederalkoxy, wie Methoxy oder Aethoxy, oder gegebenenfalls substituiertes Phenylniederalkoxy, wie gegebenenfalls substituiertes Benzyloxy. Verestertes Hydroxy bedeutet insbesondere mit einer Mineralsäure oder einer organischen Sulfonsäure verestertes Hydroxy, vor allem Halogen, wie Chlor, Brom oder Iod, Sulfonyloxy, wie gegebenenfalls durch Halogen substituiertes Niederalkansulfonyloxy, z.B. Methan- oder Trifluormethansulfonyloxy, Cycloalkansulfonyloxy, z.B. Cyclohexansulfonyloxy, oder gegebenenfalls durch Niederalkyl oder Halogen substituiertes Benzolsulfonyloxy, z.B. Benzol-, p-Bromphenyl- oder p-Toluolsulfonyloxy, ferner Niederalkanoyloxy, z.B. Acetoxy oder Pivaloyloxy. Veräthertes Mercapto ist z.B. Niederalkylthio, wie Methyl- oder Aethylthio, oder gegebenenfalls substituiertes Phenylthio, wie Phenylthio oder p-Tolylthio. Veresterte Mercaptogruppen sind beispielsweise Niederalkanoylthiogruppen, wie Acetylthio. Aminogruppen sind beispielsweise Amino-, N-Niederalkylamino-, N,N-Diniederalkylamino- oder N-Niederalkanoylaminogruppen, ferner N,N-Niederalkylen- bzw. N,N-Aza-, N,N-Oxa- oder N,N-Thianiederalkylenaminogruppen, z.B. Dimethylamino oder Diäthylamino, ferner Pyrrolidino, Piperidino, Morpholino oder Thiomorpholino, weiterhin Anilino. Ammoniumgruppen sind beispielsweise den vorstehend genannten Aminogruppen entsprechende tertiäre oder quaternäre Ammoniumgruppen, wie Triniederalkylammonio oder Pyridinio. Sulfoniumgruppen sind z.B. Diniederalkylsulfoniumgruppen, wie Dimethylsulfonium.

Gegebenenfalls funktionell abgewandeltes Oxo $G_2$ ist beispielsweise Oxo, Thioxo oder eine Gruppe =N-R′. Gruppen =N-R′ sind z.B. solche, in deren R′ Wasserstoff, Niederalkyl oder einen Acylrest, wie Niederalkanoyl, gegebenenfalls substituiertes Benzoyl, Pyridoyl oder Niederalkansulfonyl, bedeutet, beispielsweise Imino-, N-Niederalkylimino-, N-Niederalkanoylimino-,gegebenenfalls substituierte N-Benzoylimino- oder N-Niederalkansulfonyliminogruppen.

Für die Herstellung von Verbindungen I, worin $R_5$ und $R_6$ gemeinsam für eine zusätzliche Bindung stehen, oder ihrer Salze vorzugsweise verwendete Verbindungen IIa sind beispielsweise Thiophosgen ($Z_1$ = $Z_2$ = Chlor) und Kohlendisulfid (Schwefelkohlenstoff; $Z_1$ und $Z_2$ = Thiooxo), die zu Verbindungen I,

worin L für ein Schwefelatom steht, oder deren Salzen führen, und Phosgen ($Z_1$ = $Z_2$ = Chlor), das zu Verbindungen I, worin L für ein Sauerstoffatom steht, oder deren Salzen führt.

Für die Herstellung von Verbindungen I, worin $R_5$ Wasserstoff und $R_6$ Halogenmethyl bedeutet, oder ihrer Salze vorzugsweise verwendete Verbindungen IIa sind beispielsweise Halogenessigsäuren ($Z_1$ = Hydroxy; $Z_2$ = Halogenmethyl; L = Sauerstoffatom) und entsprechende reaktionsfähige Halogenessigsäurederivate ($Z_1$ z.B. = Halogen, Niederalkoxy oder Sulfonyloxy).

Die Umsetzung einer Verbindung II oder eines Salzes davon mit einer Verbindung IIa wird in üblicher Weise durchgeführt, beispielsweise gegebenenfalls in Gegenwart eines Kondensationsmittels, wie einer geeigneten Base, bei der Umsetzung mit Verbindungen IIa, worin $Z_1$ und $Z_2$ gemeinsam Thioxo bedeuten, gegebenenfalls in Gegenwart eines schwefelbindenden Mittels, bei der Umsetzung mit Verbindungen IIa, worin $Z_1$ für $G_1$ steht, $Z_2$ Halogenmethyl ist und L ein Sauerstoffatom darstellt, gegebenenfalls in Gegenwart eines wasserbindenden Mittels, in Ab- oder üblicherweise in Anwesenheit eines geeigneten inerten Lösungs- oder Verdünnungsmittels oder eines Gemisches derselben und je nach Bedarf unter Kühlen, bei Raumtemperatur oder unter Erwärmen, z.B. in einem Temperaturbereich von etwa -80 °C bis etwa +200 °C, vorzugsweise von etwa -20 °C bis etwa +150 °C, und, falls erforderlich, in einem geschlossenen Gefäss, unter Druck und/oder unter Inertgas, wie Stickstoff.

Als Basen kommen beispielsweise Alkalimetallhydroxide, -hydride, -amide, -alkanolate, -carbonate, -triphenylmethylide, -diniederalkylamide, -aminoniederalkylamide und -niederalkylsilylamide, Naphthalinamine, Niederalkylamino, basische Heterocyclen, Ammoniumhydroxide sowie carbocyclische Amine in Frage. Beispielhaft seien Natriumhydroxid, -hydrid, -amid, -äthylat, -carbonat, Kalium-tert-butylat, -carbonat, Lithiumtriphenylmethylid, Lithium-diisopropylamid, Kalium-3-(aminopropyl)-amid, -bis-(trimethylsilyl)-amid, Dimethylaminonaphthalin, Di und Triäthylamin, Pyridin, Benzyl-trimethyl-ammoniumhydroxid, 1,5-Diaza-bicyclo[4.3.0]non-5-en (DBN) sowie 1,5-Diaza-bicyclo[5.4.0]undec-5-en (DBU) genannt.

Geeignete schwefelbindende Mittel bei der Umsetzung mit Verbindungen IIa, worin $Z_1$ und $Z_2$ gemeinsam Thioxo bedeuten, sind z.B. Oxide des Phosphors, wie Tetraphosphordekaoxid, Carbodiimide, wie N,N′-Dicyclohexylcarbodiimid, und Derivate der Kohlensäure, wie Kohlensäureester, z.B. Halogenkohlensäureniederalkylester, wie Chlorkohlensäureäthylester.

Geeignete wasserbindende Mittel bei der Umsetzung mit Verbindungen IIa, worin $Z_1$ für $G_1$ steht, $Z_2$ Halogenmethyl ist und L ein Sauerstoffatom darstellt, sind z.B. Oxide des Phosphors, wie Tetraphosphordekaoxid, und Carbodiimide, wie N,N′-Dicyclohexylcarbodiimid oder N-[3-(N,N-Dimethylamino)propyl]-N′-äthylcarbodiimid und dessen Säureadditionssalze , z.B. das Hydrochlorid.

Als inerte Lösungs- oder Verdünnungsmittel sind beispielsweise Wasser und, auch in Form von Gemischen mit Wasser, cyclische Aether, gegebenenfalls halogenierte aromatischer Kohlenwasserstoffe, halogenierte Niederalkane, N,N-Diniederalkylniederalkansäureamide, Phosphorsäureniederalkylamide, Diniederalkylsulfoxide, basische Heterocyclen und Niederalkanole, wie Tetrahydrofuran, Dioxan, Benzol, Toluol, Xylol, Chlorbenzol, 1,2-Dichlorbenzol, Trichlormethan, N,N-Dimethylformamid, Hexamethylphosphorsäuretriamid, Dimethylsulfoxid, Pyridin, N-Methylmorpholin, Methanol und Aethanol, zu nennen.

Das Ausgangsmaterial II oder ein Salz davon lässt sich in Analogie zu bekannten Methoden herstellen, beispielsweise durch Hydrolyse, d.h. Umsetzung mit Wasser, einer Verbindung der Formel

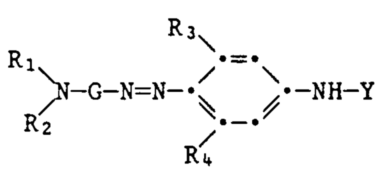

(III),

worin Y für einen Acylrest steht, oder eines Salzes davon.

Acylreste Y sind beispielsweise von einer organischen Carbon- oder Sulfonsäure abgeleitete Acylreste.

Von einer organischen Carbonsäure abgeleitetes Acyl ist beispielsweise der Rest einer aliphatischen oder monocyclisch-aromatischen Carbonsäure, wie Niederalkanoyl oder gegebenenfalls substituiertes Benzoyl, ferner Pyridoyl.

Von einer organischen Sulfonsäure abgeleitetes Acyl ist beispielsweise Niederalkansulfonyl.

Niederalkanoyl ist z.B. $C_2$-$C_5$ Alkanoyl, wie Acetyl, Propionyl, Butyryl, Isobutyryl oder Pivaloyl.

Gegebenenfalls substituiertes Benzoyl ist z.B. Benzoyl, p-Chlorbenzoyl oder p-Nitrobenzoyl.

Niederalkansulfonyl ist z.B. $C_1$-$C_4$-Alkansulfonyl, wie Methan- oder Aethansulfonyl.

Die Hydrolyse einer Verbindung III oder eines Salzes davon wird in üblicher Weise durchgeführt, beispielsweise in Gegenwart eines Hydrolysemittels sowie gegebenenfalls in Ab- oder üblicherweise in

Anwesenheit eines geeigneten inerten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80 °C bis etwa +200 °C, vorzugsweise von etwa -20 °C bis etwa +150 °C, und, falls erforderlich, in einem geschlossenen Gefäss, unter Druck und/oder unter Inertgas, wie Stickstoff, arbeitet.

Geeignete Hydrolysemittel sind z.B. Säuren oder Basen. Als Säuren kommen beispielsweise anorganische oder organische Protonsäuren, wie Mineralsäuren, z.B. Schwefelsäure oder Halogenwasserstoffsäuren, beispielsweise Chlorwasserstoffsäure, wie Sulfonsäuren, z.B. Niederalkan- oder gegebenenfalls substituierte Benzolsulfonsäuren, beispielsweise Methan- oder p-Toluolsulfonsäure, oder wie Carbonsäuren, z.B. Niederalkancarbonsäuren, beispielsweise Essigsäure, in Frage, während als Basen beispielsweise die vorstehend genannten verwendet werden können, insbesondere Natrium- oder Kaliumhydroxid.

Als inerte Lösungs- oder Verdünnungsmittel sind insbesondere die vorstehend erwähnten zu nennen, vor allem Wasser und wässrige Niederalkanole, wie wässriges Methanol oder Aethanol.

Das Ausgangsmaterial III oder ein Salz davon kann in Analogie zu bekannten Methoden hergestellt werden, beispielsweise durch Umsetzung eines Salzes der Formel

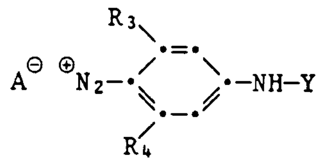 (IV),

worin A für das Anion einer Protonensäure steht, mit einer Verbindung der Formel

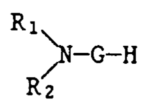 (IVa)

oder einem Salz davon.

Anionen A von Protonensäuren in Salzen IV sind beispielsweise Anionen der vorstehend für die Bildung von Säureadditionssalzen von Verbindungen I genannten Säuren, insbesondere Anionen von starken anorganischen Protonensäuren, wie Anionen von Mineralsäuren, z.B. Schwefelsäure, einer Phosphorsäure oder einer Halogenwasserstoffsäure, oder von Tetrafluoroborsäure, oder Anionen von starken organischen Carbonsäuren, wie Niederalkancarbonsäuren, z.B. Ameisen- oder Essigsäure, beispielsweise das Sulfat-, Phosphat-, Chlorid-, Bromid-, Tetrafluoroborat- oder Acetation.

Die Umsetzung eines Salzes IV mit einer Verbindung IVa oder einem Salz davon erfolgt in Analogie zu bekannten Verfahrensweisen unter den üblichen Reaktionsbedingungen, beispielsweise in einem inerten Lösungs- oder Verdünnungsmittel, z.B. der vorstehend angegebenen Art, vorzugsweise in Wasser, gegebenenfalls in Gegenwart eines sauren Mittels, beispielsweise in Gegenwart einer der vorstehend erwähnten Säuren, und/oder unter Kühlung, bei Raumtemperatur oder unter Erwärmen, beispielsweise im Temperaturbereich von etwa -20 bis etwa +50 °C, vorzugsweise von etwa 0 bis etwa +30 °C.

Das Ausgangsmaterial IV ist bekannt oder lässt sich in Analogie zu bekannten Methoden herstellen, beispielsweise durch Umsetzung einer Verbindung der Formel

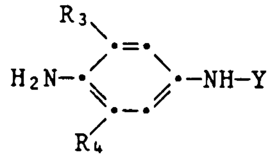 (V)

oder eines Salzes davon mit salpetriger Säure, wobei unter den üblicherweise angewendeten Reaktionsbedingungen gearbeitet wird, beispielsweise in einem Lösungs- oder Verdünnungsmittel, vorzugsweise in Wasser, und/oder unter Kühlung, bei Raumtemperatur oder unter Erwärmen, beispielsweise im Temperaturbereich von etwa -20 bis etwa +50 °C. Die salpetrige Säure wird dabei vorzugsweise <u>in situ</u>, beispielsweise

durch Umsetzung eines Alkalimetallnitrits, wie Natriumnitrit, mit einer starken Protonensäure, beispielsweise einer Halogenwasserstoffsäure, wie Chlorwasserstoffsäure, oder einer Niederalkancarbonsäure, wie Ameisensäure oder Eisessig, hergestellt.

In einer besonders bevorzugten Ausführungsform wird eine Verbindung V oder ein Salz davon wie vorstehend erläutert mit, beispielsweise in situ erzeugter, salpetriger Säure umgesetzt und das zunächst gebildete Salz IV anschliessend ohne Isolierung und/oder zusätzliche Reinigung in situ erfindungsgemäss mit einer Verbindung IVa oder einem Salz davon zu der gewünschten Verbindung III weiterumgesetzt.

Die Verbindungen IVa bzw. deren Salze sind bekannt oder in Analogie zu bekannten Methoden herstellbar.

Das Ausgangsmaterial IIa ist ebenfalls bekannt oder lässt sich in Analogie zu bekannten Methoden herstellen.

Salze von Verbindungen I können in an sich bekannter Weise hergestellt werden. So erhält man beispielsweise Säureadditionssalze von Verbindungen I durch Behandeln mit einer Säure oder einem geeigneten Ionenaustauscherreagens. Saure Verbindungen I lassen sich z.B. durch Behandlung mit einer Base oder einem geeigneten Ionenaustauscherreagens in Salze mit Basen überführen. Salze von Verbindungen I können in üblicher Weise in die freien Verbindungen I überführt werden, Säureadditionssalze z.B. durch Behandeln mit einem geeigneten basischen Mittel, Salze mit Basen beispielsweise durch Behandlung mit einem geeigneten sauren Mittel.

Je nach Verfahrensweise bzw. Reaktionsbedingungen können die Verbindungen I mit salzbildenden, insbesondere sauren, Eigenschaften in freier Form oder in Form von Salzen erhalten werden.

Infolge der engen Beziehung zwischen einer neuen Verbindung I in freier Form und in Form ihrer Salze sind im vorausgegangenen und nachfolgend unter einer freien Verbindung I oder ihren Salzen sinn- und zweckgemäss gegebenenfalls auch die entsprechenden Salze bzw. die freie Verbindung I zu verstehen.

Die neuen Verbindungen I einschliesslich ihrer Salze können auch in Form ihrer Hydrate erhalten werden oder andere, beispielsweise zur Kristallisation von in fester Form vorliegenden Verbindungen verwendete, Lösungsmittel einschliessen.

Die neuen Verbindungen I können, je nach Wahl der Ausgangsstoffe und Arbeitsweisen, in Form eines der möglichen Isomeren oder als Gemisch derselben vorliegen. Dabei sind, in Abhängigkeit von der Molekülsymmetrie, z.B. je nach Anzahl, absoluter und relativer Konfiguration der Chiralitätszentren, wie asymmetrischen C-Atome, als reine Isomere z.B. reine Enantiomere und/oder reine Diastereomere, wie reine cis/trans-Isomere oder meso-Verbindungen, erhältlich. Entsprechend können als Isomerengemische z.B. Enantiomerengemische, wie Racemate, Diastereomerengemische oder Racematgemische vorliegen.

Erhaltene Diastereomerengemische und Racematgemische können aufgrund der physikalisch-chemischen Unterschiede der Bestandteile in bekannter Weise in die reinen Diastereomeren oder Racemate aufgetrennt werden, beispielsweise durch fraktionierte Kristallisation.

Erhaltene Enantiomerengemische, wie Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Herstellungsverfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Enantiomeren verwendet oder insbesondere unter den Reaktionsbedingungen bildet.

Beim Herstellungsverfahren der vorliegenden Erfindung werden vorzugsweise solche Ausgangsstoffe verwendet, welche zu den vorstehend als besonders wertvoll geschilderten Verbindungen I führen. Neue Ausgangsstoffe, die speziell für die Herstellung der Verbindungen I entwickelt wurden, ihre Verwendung und Verfahren zu ihrer Herstellung bilden ebenfalls einen Gegenstand der Erfindung, wobei die Variablen $R_1$, $R_2$, $R_3$, $R_4$, $R_5$, $R_6$, G und L die für die jeweils bevorzugten Verbindungsgruppen der Formel I angegebenen Bedeutungen haben.

In diesem Zusammenhang sind insbesondere Verbindungen der Formel

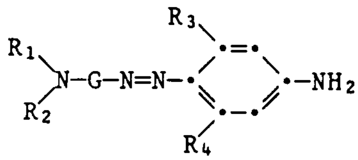

(II)

und deren Salze, für die in analoger Weise das vorstehend für Salze von Verbindungen I Gesagte gilt, zu erwähnen. Diese können in besonders vorteilhafter Weise als Ausgangsstoffe für die Herstellung von Verbindungen I oder deren Salzen, z.B. gemäss dem vorstehend beschriebenen Verfahren, Verwendung finden.

Die Erfindung betrifft dementsprechend ebenfalls Verbindungen der Formel II, worin $R_1$ Niederalkyl darstellt, $R_2$ Niederalkyl ist, $R_3$ Wasserstoff, Carboxy oder Sulfo bedeutet, $R_4$ Carboxy oder Sulfo ist und G für eine gegebenenfalls substituierte 1,4-Phenylengruppe oder eine gegebenenfalls substituierte 1,4-Naphthylengruppe steht, und ihre Salze, die Verwendung dieser Verbindungen und ihrer Salze und ein Verfahren zur Herstellung dieser Verbindungen und ihrer Salze.

Die Variablen der Formel II haben dabei beispielsweise die unter der Formel I angegebenen bevorzugten Bedeutungen.

Die Erfindung betrifft diesbezüglich in erster Linie Verbindungen der Formel II, worin $R_1$ Niederalkyl darstellt, $R_2$ Niederalkyl ist, $R_3$ Wasserstoff, Carboxy oder Sulfo bedeutet, $R_4$ Carboxy oder Sulfo ist und G für eine unsubstituierte oder durch Carboxy und/oder Sulfo substituierte 1,4-Phenylengruppe oder eine unsubstituierte oder durch Carboxy und/oder Sulfo substituierte 1,4-Naphthylengruppe steht, und ihre Salze.

Die Erfindung betrifft diesbezüglich vor allem Verbindungen der Formel II, worin $R_1$ $C_1$-$C_4$-Alkyl, wie Methyl oder Aethyl, darstellt, $R_2$ $C_1$-$C_4$-Alkyl, wie Methyl oder Aethyl, ist, $R_3$ Wasserstoff oder Sulfo bedeutet, $R_4$ Sulfo ist und G eine unsubstituierte oder durch Sulfo substituierte 1,4-Phenylengruppe darstellt, und ihre Salze.

Die Erfindung betrifft diesbezüglich in allererster Linie Verbindungen der Formel II, worin $R_1$ $C_1$-$C_4$-Alkyl, wie Methyl, darstellt, $R_2$ $C_1$-$C_4$-Alkyl, wie Methyl, ist, $R_3$ Wasserstoff bedeutet, $R_4$ für Sulfo steht und G eine unsubstituierte 1,4-Phenylengruppe darstellt, und ihre Salze.

Die Erfindung betrifft diesbezüglich namentlich die in den Beispielen genannten neuen Verbindungen der Formel II und ihre Salze.

Ebenfalls Gegenstand der vorliegenden Erfindung ist ein Verfahren zur Herstellung einer Verbindung II oder eines Salzes davon, z.B. dadurch gekennzeichnet, dass man eine Verbindung der Formel

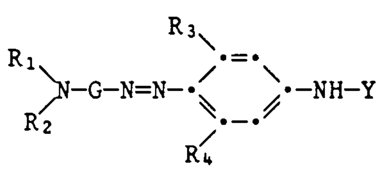

(III),

worin Y für einen Acylrest steht, oder ein Salz davon hydrolysiert, beispielsweise wie vorstehend beschrieben, und gewünschtenfalls ein verfahrensgemäss erhältliches Isomerengemisch in die Komponenten auftrennt und das gewünschte Isomere II isoliert, ein verfahrensgemäss erhältliches Enantiomeren- oder Diastereomerengemisch in die Enantiomeren oder Diastereomeren aufspaltet und das gewünschte Enantiomere oder Diastereomere isoliert und/oder eine verfahrensgemäss erhältliche freie Verbindung II in ein Salz überführt oder ein verfahrensgemäss erhältliches Salz in die freie Verbindung II oder in ein anderes Salz umwandelt.

Die Herstellung von Verbindungen III oder deren Salzen wird vorstehend beschrieben.

An verfahrensgemäss oder anderweitig erhältlichen Verbindungen II oder deren Salzen gewünschtenfalls vorzunehmende Nachoperationen sind insbesondere Enantiomeren- oder Diastereomerentrennungen und gegenseitige Umwandlungen von Salzen und freien Verbindungen II wie für die Verbindungen I angegeben, die auch analog durchgeführt werden.

Die Erfindung betrifft ebenfalls die Verwendung von Verbindungen II oder deren Salzen als Ausgangsstoffe für die Herstellung von Verbindungen I oder deren Salzen.

Es ist möglich, das vorstehend beschriebene optimierte Verfahren für die chemische Modifizierung von Proteinen und für die Analyse der Primärstruktur von solchen chemisch modifizierten Proteinen, d.h. die Sequenz, bestehend aus der chemischen Modifizierung mittels einer Verbindung I oder eines Salzes davon, aus einer gegebenenfalls vorzunehmenden Denaturierung, aus der Proteasebehandlung und aus der HPLC, auch automatisch durchzuführen, wobei eine speziell zu diesem Zweck hergestellte Vorrichtung verwendet wird.

Der Aufbau dieser Vorrichtung ist schematisch in Abbildung 1 dargestellt. Die erfindungsgemässe Vorrichtung besteht aus den Komponenten 1 bis 28. Die Vorrichtung enthält eine Spritzenpumpe (Einspritzeinrichtung, "syringe pump") 24, welche dazu dient, eine Probe des Proteins, welches mittels einer Verbindung 1 oder eines Salzes davon chemisch modifiziert werden soll, Lösungsmittel, Reagentien und/oder Katalysatoren, welche für die erwähnten Sequenzteilschritte der chemischen Modifizierung, der gegebenenfalls vorzunehmenden Denaturierung und der Proteasebehandlung nötig sind, Inertgas und Waschflüssigkeit durch die Verbindung 1 einzubringen in den Reaktor 15, welcher Reaktor 15 mit einem Schraubdeckel 16 versehen und in einem Heizmantel 14 angeordnet ist, eine Vakuumpumpe 25, die mittels der Verbindung 2 die Evakuierung des Reaktors 15 zum Zwecke der Konzentration des Reaktorinhalts ermöglicht, eine Verbindung 3, die dazu dient, mittels eines schwachen Inertgasüberdruckes (das Inertgas wird mittels der Spritzenpumpe 24 durch die Verbindung 1 herangeführt) den Inhalt des Reaktors 15, wenn der Teilschritt der chemischen Modifizierung oder der gegebenenfalls auszuführende Teilschritt der Denaturierung ausgeführt worden ist, durch das Ventil 26, die Verbindung 5, das Ventil 27 und die Verbindung 9 zu der Entsalzungseinrichtung 19 zu überführen, oder, wenn der Teilschritt der Proteasebehandlung ausgeführt worden ist, durch das Ventil 26, die Verbindung 5, das Ventil 27 und die Verbindung 7 zu der HPLC-Komponente 21, an welche eine Zeichenkomponente ("Plotter") 22 und, mittels der Verbindung 8, ein Fraktionssammler 20 angeschlossen sind, zu überführen, oder die dazu dient, die Waschflüssigkeit, die im Reaktor 15 enthalten ist, mittels eines schwachen Inertgasüberdruckes durch das Ventil 26 und die Verbindung 11 in den Abfallflüssigkeitsbehälter 17 zu überführen, eine Pumpe 23, die Laufmittel durch die Verbindung 6, das Ventil 27 und die Verbindung 9 zur Entsalzungseinrichtung 19 fördert, eine Verbindung 10, die dazu dient, das aus der Entsalzungseinrichtung 19 austretende Eluat zu dem Detektor 18 zu leiten, welcher dazu dient, das Ventil 28 in der Weise einzustellen, dass das aus 19 austretende Eluat, wenn es das im Schritt der chemischen Modifizierung hergestellte chemisch modifizierte Protein oder das gegebenenfalls im Denaturierungsschritt hergestellte denaturierte chemisch modifizierte Protein enthält, durch die Verbindung 4 in den Reaktor 15 gelangt und, wenn es kein solches chemisch modifiziertes Protein oder denaturiertes chemisch modifiziertes Protein enthält, durch die Verbindung 12 in den Abfallflüssigkeitsbehälter 17 gelangt, und eine Verbindung 13, die den Transport des Eluats vom Detektor 18 zum Ventil 28 ermöglicht.

Der Reaktor 15 ist in Grösse und Form mit einem 1,5 ml-Eppendorf-Röhrchen vergleichbar; er kann aus irgendeinem inerten Material bestehen, zum Beispiel aus Glas, aus möglichst weitgehend inertem Kunststoff, wie Polytetrafluoräthylen, oder aus Edelstahl, vorzugsweise aus einem transparenten Material, z.B. aus Glas oder einem transparenten, möglichst weitgehend inerten Kunststoff. Die chemische Modifizierung, die gegebenenfalls vorzunehmende Denaturierung und die Proteasebehandlung werden in dem spitz zulaufenden unteren Bereich des Reaktors 15 durchgeführt; das gesamte Reaktionsvolumen beträgt dabei jeweils von etwa 100 bis 300 µl. Der Reaktor 15 kann durch den Schraubdeckel 16, welcher vorzugwseise aus dem gleichen Material besteht wie der Reaktor 15, verschlossen werden. Die Verbindung 3 sollte so nahe wie möglich an den Boden des Reaktors 15 heranreichen, um eine praktisch vollständige Leerung des Reaktors zu ermöglichen.

Durch die Spritzenpumpe 24 können das Edukt (das Protein, das chemisch modifiziert werden soll), die notwendigen Lösungsmittel, Reagentien, z.B. die Verbindung I, die bei dem Schritt der chemischen Modifizierung verwendet wird, oder ein Salz davon, und/oder Katalysatoren, des weiteren Inertgas und Waschflüssigkeit in den Reaktor 15 eingebracht werden. Die Spritzenpumpe 24 ist ein handelsübliches Produkt, das es aufgrund seiner Konstruktion erlaubt, die Art und die Menge des Agens, das in den Reaktor 15 eingebracht werden soll, jederzeit unabhängig auszuwählen. Mittels eines in 24 eingebauten Ventils kann die in den Reaktor 15 führende Verbindung 1 jederzeit verschlossen werden, z.B. dann, wenn die gewünschte Menge eines bestimmten Agens in den Reaktor 15 gepumpt worden ist. Die Mengen, die in einem einzelnen Schritt in den Reaktor 15 gepumpt werden können, sind innerhalb eines weiten Volumenbereiches variabel und reichen z.B. von etwa 1 µl bis etwa 200 µl.

Als Vakuumpumpe 25 kann jede handelsübliche Pumpe verwendet werden, vorzugsweise eine Oelpumpe. Die Entsalzungseinrichtung 19 dient dazu, überschüssige Reagentien und Nebenprodukte zu entfernen, welche nach der Stufe der chemischen Modifizierung und nach der gegebenenfalls ausgeführten Stufe der Denaturierung im Reaktionsgemisch enthalten sind. Als Entsaltzungseinrichtung 19 können üblicherweise

verwendete chromatographische Trennvorrichtungen eingesetzt werden, z.B. solche, die für die Säulnchromatographie, wie die molekulare Ausschlusschromatographie, z.B. die Gelchromatographie, Verwendung finden. Der Detektor 18 arbeitet vorzugsweise auf der Basis des Lichtabsorptionsverhaltens, z.B. bei Wellenlängen des ultravioletten oder insbesondere des sichtbaren Lichts, der Moleküle. Typische Wellenlängen sind jene, die vorstehend für den Detektor des HPLC-Systems angegeben sind. Als Heizmantel 14, Abfallflüssigkeitsbehälter 17, Fraktionssammler 20, HPLC-Komponente 21, Zeichenkomponente 22 und Pumpe 23, welche das Laufmittel für die Entsalzungseinrichtung 19 fördert, können geeignete handelsübliche Komponenten verwendet werden.

Das Ventil 26 kann einerseits die Verbindungen 3 und 5 öffnen und gleichzeitig die Verbindung 11 verschliessen. Andererseits kann 26 die Verbindungen 3 und 11 öffnen und gleichzeitig die Verbindung 5 schliessen. Das Ventil 27 kann entweder 5 und 9 öffnen und gleichzeitig 6 und 7 verschliessen oder 5 und 7 öffnen und gleichzeitig 6 und 9 verschliessen oder 6 und 9 öffnen und gleichzeitig 5 und 7 verschliessen. Das Ventil 28 kann entweder 4 und 13 öffnen und gleichzeitig 12 verschliessen oder 12 und 13 öffnen und gleichzeitig 4 verschliessen. Handelsübliche Ventile geeigneten Typs können verwendet werden.

Die Verbindungen 1 und 3 bis 13 können aus irgend einem inerten Material bestehen, z.B. aus Glas oder möglichst weitgehend inertem Kunststoff, wie Polytetrafluoräthylen, vorzugsweise aus einem transparenten Material, z.B. aus Glas oder einem transparenten, möglichst weitgehend inerten Kunststoff. Die Verbindung 2 besteht aus vakuumbeständigem Schlauch.

In einzelnen wird die Reaktionssequenz wie folgt durchgeführt: Eine Lösung des Proteins, das durch eine Verbindung I oder ein Salz davon chemisch modifiziert werden soll, wird mittels der Spritzenpumpe 24 durch die Verbindung 1 in den Reaktor 15 eingebracht. Anschliessend wird eine Lösung des Reagens (einer Verbindung 1 oder eines Salzes davon) in gleicher Weise in 15 eingebracht. Nach Beendigung der chemischen Modifizierung wird ein schwacher Inertgasüberdruck, z.B. von Argon, in Reaktor 15 aufgebaut (das Inertgas wird mittels der Spritzenpumpe 24 durch die Verbindung 1 zugeführt). Der Ueberdruck bewirkt, dass der Inhalt des Reaktors 15 via 3, 26, 5, 27 und 9 zur Entsalzungseinrichtung 19 transferiert wird. Die Ventile 26 und 27 werden dazu entsprechend eingestellt. Die Verbindung 4 ist mittels des Ventils 28 verschlossen. Wenn der gesamte Reaktorinhalt zur Entsalzungseinrichtung 19 transferiert worden ist, wird der Ueberdruck abgebaut. Die Probe wird dann, um sie zu reinigen, durch die Entsalzungseinrichtung 19 geschickt, wobei die Pumpe 23 via 6, 27 und 9 (das Ventil 27 wird entsprechend eingestellt) das Laufmittel heranführt. Mittels 10, 18, 13, 28 und 4 wird, bei entsprechender Einstellung des Ventils 28, das gereinigte chemisch modifizierte Protein in den Reaktor 15 zurückgeführt. Der Detektor 18 dient dazu, das Ventil 28 in der Weise einzustellen, dass nur die Fraktion des Eluates, die das gewünschte Produkt enthält, in den Reaktor 15 überführt wird, hingegen die anderen Fraktionen direkt via 12 in den Abfallflüssigkeitsbehälter 17 geleitet werden. Der Reaktorinhalt (Eluat, welches das gereinigte chemisch modifizierte Protein enthält) wird dann aufkonzentriert, indem über die Verbindung 2 mittels der Vakuumpumpe 25 Vakuum angelegt wird. Während der Evakuierung ist die Verbindung 1 mittels des in der Spritzenpumpe 24 eingebauten Ventils verschlossen. Ebenso sind die Verbindungen 3 bzw. 5 (mittels des Ventils 27) sowie 4 (mittels des Ventils 28) verschlossen.

Wenn ein Denaturierungsschritt ausgeführt werden soll, so erfolgt dieser in analoger Weise wie vorstehend für den Schritt der chemischen Modifizierung beschrieben, wobei geeignete Reagentien und Lösungsmittel eingesetzt werden. Diesem Denaturierungsschritt folgt dann wiederum eine Reingigungsstufe, bei der die Entsalzungseinrichtung 19 in analoger Weise gebraucht wird. Das resultierende Eluat wird wiederum in den Reaktor 15 überführt und dort aufkonzentriert.

Schliesslich wird in analoger Weise wie für den Schritt der chemischen Modifizierung beschrieben der Schritt der Proteasebehandlung im Reaktor 15 ausgeführt, wobei wiederum geeignete Reagentien und Lösungsmittel eingesetzt werden. Nach Beendigung der Proteasebehandlung wird der Reaktorinhalt via 3, 26, 5, 27 und 7 (die Ventile 26 und 27 werden entsprechen eingestellt) zur HPLC-Komponente 21 transferiert, wobei in analoger Weise wie vorstehend beschrieben verfahren wird, d.h. unter Anwendung eines schwachen Inertgasüberdrucks. Nach Durchführung der HPLC wird das Chromatogramm auf der Zeichenkomponente 22 ausgegeben. Die nach der HPLC anfallenden Fraktionen können mittels des Fraktionssammlers 20 gesammelt werden.

Die Reaktionsbedingungen (Temperatur, Volumen, Reaktonszeit, etc.) und der Gradient der HPLC werden durch ein zuvor gewähltes Programm kontrolliert. In den beschriebenen Reaktionsschritten der Sequenz nötige Katalysatoren oder andere Hilfsmittel können mittels der Spritzenpumpe 24 via 1 in den Reaktor 15 eingebracht werden.

Die Vorrichtung wird in der Weise benutzt, dass einerseits die Entsalzungseinrichtung 19 kontinuierlich mit Laufmittel gewaschen wird (welches von der Pumpe 23 herangeführt wird), solange eine Reaktion (chemische Modifizierung, gegebenenfalls Denaturierung oder Proteasebehandlung) im Reaktor 15 stattfindet, und dass andererseits der Reaktor 15 mit Waschflüssigkeit (zugeführt mittels der Spritzenpumpe 24 via 1) gereinigt wird, solange die Probe (das chemisch modifizierte Protein oder gegebenenfalls das denaturierte chemisch modifzierte Protein) in der Entsalzungseinrichtung 19 gereingt wird. Die von 19 her anfallende Waschflüssigkeit wird via 10, 18, 13, 28 und 12, die von 15 her anfallende Waschflüssigkeit via 3, 26 und 11 in den Abfallflüssigkeitsbehälter17 transferiert, wobei jeweils analog wie vorstehend für den Transfer von Produkten und Eluaten beschrieben verfahren wird.

Die Erfindung betrifft ebenfalls eine Vorrichtung der zuvor beschriebenen Art, die speziell zu dem Zweck hergestellt ist, die Sequenz, bestehend aus der chemischen Modifizierung eines Proteins mittels einer Verbindung I oder eines Salzes davon, aus einer gegebenenfalls vorzunehmenden Denaturierung des chemisch modifizierten Proteins, aus der Proteasebehandlung des chemisch modifzizierten und gegebenenfalls denaturierten Proteins und aus der HPLC des nach der Proteasebehandlung erhaltenen Peptidgemisches, automatisch durchzuführen.

Die Erfindung betrifft ebenfalls ein Verfahren zur Herstellung dieser Vorrichtung, das dadurch gekennzeichnet ist, dass die zuvor beschriebenen Komponenten der Vorrichtung in der zuvor beschriebenen Weise kombiniert werden, um die gewünschte Vorrichtung zu ergeben.

Die Erfindung betrifft ebenfalls ein Verfahren zur automatischen chemischen Modifizierung eines Proteins und zur automatischen Analyse der Primärstruktur des chemisch modifizierten Proteins, bestehend aus der zuvor beschriebenen Sequenz, welches Verfahren dadurch gekennzeichnet ist, dass das Protein in der zuvor beschriebenen Vorrichtung bearbeitet wird.

Die Erfindung betrifft ebenfalls die Verwendung der zuvor beschriebenen Vorrichtung für die automatische Durchführung der zuvor beschriebenen Sequenz und die Verwendung einer Verbindung I oder eines Salzes davon als Reagens in dem in der zuvor beschriebenen Vorrichtung durchgeführten Schritt der chemischen Modifizierung.

Die nachfolgenden Beispiele sind ebenfalls ein Gegenstand der Erfindung und illustrieren die vorstehend beschriebene Erfindung; sie sollen jedoch diese in ihrem Umfang in keiner Weise einschränken. Temperaturen sind in Celsiusgraden angegeben. HPLC steht für hochauflösende Flüssigkeitschromatographie. Lys bedeutet Lysin. Val steht für Valin.

<u>Beispiel 1:</u> 1 g (3,1 mmol) 4-Amino-4'-(N,N-dimethylamino)-azobenzol-2-sulfonsäure wird mit 25 ml 0,24 M-Natriumcarbonatlösung und 0.91 g (7,9 mmol; 0,6 ml) Thiophosgen versetzt. Das Reaktionsgemisch wird 30 Minuten bei 70° gerührt. Nach dem Abkühlen auf Raumtemperatur wird das ausgefallene Produkt mittels einer Glasfritte abfiltriert und nacheinander gründlich mit 1 N-Salzsäure, Toluol und nochmals 1 N-Salzsäure gewaschen. Die schwarzen Kristalle werden sodann bei Raumtemperatur im Vakuum getrocknet. Man erhält die 4'-(N,N-Dimethylamino)-4-isothiocyanato-azobenzol-2-sulfonsäure. Das Produkt zersetzt sich beim Erwärmen und hat keinen definierten Schmelzpunkt; im Infrarotspektrum findet man eine starke Bande bei 2110 $cm^{-1}$, hervorgerufen durch die asymmetrische N=C=S-Streckschwingung.

Die 4-Amino-4'-(N,N-dimethylamino)-azobenzol-2-sulfonsäure kann man z.B. folgendermassen erhalten:

3,5 g (15 mmol) 5-(N-Acetylamino)-2-amino-benzolsulfonsäure werden mit 0,9 g (8,5 mmol) wasserfreiem Natriumcarbonat und 38 ml Wasser versetzt. Das Reaktionsgemisch wird so lange gerührt, bis eine klare Lösung entstanden ist. Diese Lösung wird anschliessend mittels eines Eisbades auf +10° gekühlt. Nach Zugabe von 1,22 g (17,7 mmol) festem Natriumnitrit wird weitere 2 Minuten gerührt. Sodann wird das Reaktionsgemisch auf eine Mischung aus 3,5 ml konzentrierter Salzsäure und 25 g zerstossenem Eis gegossen. Man lässt weitere 30 Minuten bei 0° rühren und versetzt das Reaktionsgemisch sodann mit einer Lösung von 2,01 g (16,6 mmol; 2,1 ml) N,N-Dimethylanilin in 3 ml Eisessig. Während 3 bis 4 Stunden lässt man das Reaktionsgemisch auf Raumtemperatur auftauen. Das ausgefallene Produkt wird abfiltriert und über Nacht bei Raumtemperatur im Vakuum getrocknet. Man erhält auf diese Weise die 4-(N-Acetylamino)-4'-(N,N-dimethylamino)-azobenzol-2-sulfonsäure in Form von schwarzen bis purpurfarbenen Kristallen, die sich beim Erwärmen zersetzen und keinen definierten Schmelzpunkt aufweisen.

2 g (5,5 mmol) 4-(N-Acetylamino)-4'-(N,N-dimethylamino)-azobenzol-2-sulfonsäure werden in 8 ml Aethanol gelöst. Nach Versetzen mit 4 ml 11 N-Natriumhydroxidlösung wird das Gemisch 60 Minuten bei 90° gerührt. Man lässt anschliessend auf Raumtemperatur abkühlen und gibt dann 5 ml 11,5 N-Salzsäure zu. Das Reaktionsgemisch wird sodann über Nacht bei +4° belassen. Das ausgefallene Produkt wird abfiltriert und gründlich mit 1 N-Salzsäure gewaschen. Nach Trocknung im Vakuum bei Raumtemperatur erhält man die 4-Amino-4'-(N,N-dimethylamino)-azobenzol-2-sulfonsäure in Form von schwarzen bis purpurfarbenen Kristallen, die sich beim Erwärmen zersetzen und keinen definierten Schmelzpunkt aufweisen.

Beispiel 2: 0,67 g (2,1 mmol) 4-Amino-4′-(N,N-dimethylamino)-azobenzol-2-sulfonsäure (Beispiel 1) werden in 5 ml absolutem Pyridin gelöst. Diese Lösung wird unter Rühren und Eis-Kochsalz-Kühlung zu einem Gemisch aus 424 mg (2,1 mmol) N,N′-Dicyclohexylcarbodiimid, 5 ml wasserfreiem Pyridin und 1,26 g (16,6 mmol; 1 ml) Kohlendisulfid (Schwefelkohlenstoff) zugetropft. Unter Beibehaltung der Kühlung wird das Reaktionsgemisch 3 bis 4 Stunden nachgerührt. Anschliessend lässt man das Gemisch 17 Stunden bei Raumtemperatur stehen. Sodann werden das Pyridin und das überschüssige Kohlendisulfid unter vermindertem Druck so weit wie möglich entfernt. Nach Säulenchromatographie des Rückstandes an Kieselgel (150 bis 200 mesh) mit Benzol als Laufmittel erhält man die 4′-(N,N-Dimethylamino)-4-isothiocyanato-azobenzol-2-sulfonsäure, die beim Eindampfen des Eluates auskristallisiert.

Beispiel 3: 0,67 g (2,1 mmol) 4-Amino-4′-(N,N-dimethylamino)-azobenzol-2-sulfonsäure (Beispiel 1) werden in 5 ml absolutem Pyridin gelöst. Die Lösung wird unter Rühren und Eiskühlung mit 1,26 g (16,6 mmol; 1 ml) Kohlendisulfid (Schwefelkohlenstoff) und 0,22 g (2,2 mmol; 0,3 ml) Tiäthylamin versetzt. Unter Beibehaltung der Kühlung wird das Reaktionsgemisch 3 bis 4 Stunden nachgerührt. Anschliessend lässt man das Gemisch 17 Stunden bei Raumtemperatur stehen und dampft es dann zur Trockne ein. Das als Rückstand hinterbleibende Triäthylammoniumdithiocarbamat wird über Nacht im Vakuum getrocknet und anschliessend in 12 ml Trichlormethan gelöst. Die Lösung wird unter Eiskühlung mit 0,22 g (2,2 mmol; 0,3 ml) Triäthylamin versetzt. Zu diesem Gemisch werden unter Rühren und Eiskühlung innerhalb von 4 Minuten 0,34 g (3,1 mmol; 0,3 ml) Chlorkohlensäureäthylester zugetropft. Man lässt das Reaktionsgemisch 3 bis 4 Stunden unter Beibehaltung der Kühlung stehen und dampft es dann unter vermindertem Druck zur Trockne ein. Das als Rückstand hinterbleibende Rohprodukt wird wie in Beispiel 2 beschrieben säulenchromatographisch gereinigt. Man erhält so die 4′-(N,N-Dimethylamino)-4-isothiocyanatoazobenzol-2-sulfonsäure.

Beispiel 4: In analoger Weise wie in den Beispielen 1 bis 3 beschrieben kann man, ausgehend von 4-Amino-4′-(N,N-dimethylamino)-azobenzol-2,6-disulfonsäure bzw. 4-(N,N-Dimethylamino)naphthalin-1-azo-(4′-aminobenzol-2′-sulfonsäure, auch
die 4′-(N,N-Dimethylamino)-4-isothiocyanato-azobenzol-2,6-disulfonsäure bzw.
die 4-(N,N-Dimethylamino)naphthalin-1-azo-(4′-isothiocyanatobenzol-2′-sulfonsäure) erhalten.

Das Ausgangsmaterial kann jeweils in analoger Weise wie in Beispiel 1 beschrieben, ausgehend von 4-(N-Acetylamino)-4′-(N,N-dimethylamino)-azobenzol-2,6-disulfonsäure bzw. 4-(N,N-Dimethylamino)-naphthalin-1-azo-[4′-(N-acetylamino)benzol-2′-sulfonsäure], erhalten werden.

Beispiel 5: Mittels Eis/Kochsalz-Kühlung werden 20 ml Chlorbenzol auf 0° gekühlt. In das gekühlte Chlorbenzol werden unter Beachtung der üblichen Vorsichtsmassnahmen 1,1 g (11,1 mmol) Phosgen einkondensiert. Anschliessend wird unter Beibehaltung der Kühlung und kräftigem Rühren eine Lösung von 1,79 g (5,6 mmol) 4-Amino-4′-(N,N-dimethylamino)-azobenzol-2-sulfonsäure (Beispiel 1) in 30 ml Chlorbenzol so schnell zugetropft, dass die Temperatur des Reaktionsgemisches nicht über +25° steigt. Nach Beendigung des Zutropfens wird das Kühlbad durch ein Heizbad ersetzt, mittels dessen das Reaktionsgemisch unter ständigem Weiterrühren innert 90 Minuten auf 130° aufgeheizt wird. Sobald das Reaktionsgemisch dabei eine Temperatur von 75° erreicht, wird langsam so lange weiteres Phosgen eingeleitet, bis eine klare Lösung erhalten wird. Anschliessend wird das Reaktionsgemisch unter Durchleiten von Stickstoff so lange unter Rückfluss erhitzt, bis im abgehenden Gasstrom kein Phosgen mehr nachweisbar ist (ca. zwei Stunden). Nach dem Abkühlen auf Raumtemperatur wird das Reaktionsgemisch unter vermindertem Druck so weit wie möglich eingeengt. Die Säulenchromatographie des Rückstandes an Kieselgel (150 bis 200 mesh) mit Benzol als Laufmittel liefert die 4′-(N,N-Dimethylamino)-4-isocyanato-azobenzol-2-sulfonsäure, welche beim Eindampfen des Eluates auskristallisiert.

Beispiel 6: In analoger Weise wie in Beispiel 5 beschrieben kann man, ausgehend von 4-Amino-4′-(N,N-dimethylamino)-azobenzol-2,6-disulfonsäure bzw. 4-(N,N-Dimethylamino)naphthalin-1-azo-(4′-aminobenzol-2′-sulfonsäure, auch
die 4′-(N,N-Dimethylamino)-4-isocyanato-azobenzol-2,6-disulfonsäure bzw. die 4-(N,N-Dimethylamino)-naphthalin-1-azo-(4′-isocyanatobenzol-2′-sulfonsäure) erhalten.

Das Ausgangsmaterial kann jeweils in analoger Weise wie in Beispiel 4 beschrieben erhalten werden.

Beispiel 7: 1,8 g (9,7 mmol) Iodessigsäure und 1,2 g (6,3 mmol) N-[3-(N,N-Dimethylamino)propyl]-N′-äthyl-carbodiimid-hydrochlorid werden in 9 ml Wasser gelöst. Diese Lösung wird unverzüglich unter Rühren mit einer Lösung von 0,5 g (1,6 mmol) 4-Amino-4′-(N,N-dimethylamino)-azobenzol-2-sulfonsäure (Beispiel 1) in 7,6 ml einer Pufferlösung, die einen pH-Wert von 9 aufweist und aus 50 mM-Natriumhydrogencarbonatlösung und 50 mM-Natriumcarbonatlösung besteht, vermischt. Das Gemisch wird tropfenweise mit 0,4 ml 11 N-Natronlauge versetzt und sodann 10 Minuten bei Raumtemperatur gerührt. Anschliessend wird so lange 1 N-Salzsäure tropfenweise zugegeben, bis das Gemisch purpurfarben wird. Das dabei ausfallende Produkt wird abfiltriert, gründlich mit 100 ml 0,1 N-Salzsäure gewaschen und im Vakuum getrocknet. Das getrocknete Rohprodukt wird in einem Gemisch aus 16 ml N,N-Dimethylformamid und 0,4 ml Triäthylamin gelöst. Nach erneuter Ausfällung mit 1 N-Salzsäure in der zuvor beschriebenen Weise, Filtration und Trocknung des Filterinhalts im Vakuum erhält man die gewünschte reine 4′-(N,N-Dimethylamino)-4-(N-iodacetyl)amino-azobenzol-2-sulfonsäure, die sich beim Erwärmen zersetzt und keinen definierten Schmelzpunkt besitzt.

Beispiel 8: In analoger Weise wie in Beispiel 7 beschrieben kann man, ausgehend von 4-Amino-4′-(N,N-dimethylamino)-azobenzol-2,6-disulfonsäure bzw. 4-(N,N-Dimethylamino)naphthalin-1-azo-(4′-aminobenzol-2′-sulfonsäure, auch die 4′-(N,N-Dimethylamino)-4-(N-iodacetyl)amino-azobenzol-2,6-disulfonsäure bzw. die 4-(N,N-Dimethylamino)naphthalin-1-azo-[4′-(N-iodacetyl)aminobenzol-2′-sulfonsäure] erhalten.

Das Ausgangsmaterial kann jeweils in analoger Weise wie in Beispiel 4 beschrieben erhalten werden.

Beispiel 9: Monoklonale Antikörper, die spezifisch gegen Eglin C wirken, werden bei Raumtemperatur während 18 Stunden in einer 50 mM-Natriumhydrogencarbonatlösung durch Einwirkung einer 2 mM Lösung von 4′-(N,N-Dimethylamino)-4-isothiocyanato-azobenzol-2-sulfonsäure chemisch modifiziert. Die anschliessende Strukturanalyse zeigt, das jede Antikörpermolekel durch 30 Molekeln 4′-(N,N-Dimethylamino)-4-isothiocyanato-azobenzol-2-sulfonsäure chemisch modifiziert worden ist. Bei diesem Modifizierungsgrad besitzen die Antikörper im Vergleich zu den nicht-modifizierten Antikörpern eine unveränderte Spezifität gegen und Affinität zu rekombinantem Eglin C. Diese chemisch modifizierten und dabei gleichzeitig angefärbten Antikörper können dazu verwendet werden, mit Hilfe des Sandwich-Testes das Vorhandensein von Eglin C nachzuweisen. Hierbei können Konzentrationen, die 1 µg/ml oder mehr betragen, mit dem unbewaffneten Auge erkannt werden.

Beispiel 10: Hirudin, ein spezifischer Inhibitor von Thrombin, wird bei 37° während 7 Stunden in einer 50 mM-Natriumhydrogencarbonatlösung durch Einwirkung einer 2 mM Lösung von 4′-(N,N-Dimethylamino)-4-isothiocyanato-azobenzol-2-sulfonsäure chemisch modifiziert. Das so modifizierte Hirudin wird der Gelchromatographie an einer Sephadex-G-25-Säule unterworfen. Bei der Modifizierung des Inhibitors werden pro mol Hirudin 1,96 mol 4′-(N,N-Dimethylamino)-4-isothiocyanato-azobenzol-2-sulfonsäure umgesetzt. Die Struktur des modifizierten Inhibitors und seine biologische Aktivität werden analysiert. Dabei werden folgende Befunde erhalten:

a) Das chemisch modifizierte Hirudin besitzt eine verringerte Inhibitor-Aktivität für Thrombin, wie aus dem 300-fach grösseren Wert der entsprechenden Dissoziationskonstante $K_i$ hervorgeht.

b) Monoklonale Antikörper gegen natives Hirudin wirken auch gegen das chemisch modifizierte Hirudin.

c) Das chemisch modifizierte Hirudin wird carboxymethyliert und mit Protease V8 behandelt. Die so erhaltenen Peptide werden der HPLC unterworfen. Bei einer Wellenlänge von 536 nm werden zwei Peptide detektiert. Aus der Strukturanalyse ergibt sich, dass diese beiden farbigen Peptide den Aminosäurebausteinen 1 bis 7 und 18 bis 37 entsprechen und dass selektiv die Bausteine Val-1 und Lys-27 durch 4'-(N,N-Dimethylamino)-4-isothiocyanato-azobenzol-2-sulfonsäure modifiziert werden.

Beispiel 11: Menschliches Antithrombin wird bei Raumtemperatur während 15 Minuten in einer 50 mM-Natriumhydrogencarbonatlösung durch Einwirkung einer 1 mM Lösung von 4'-(N,N-Dimethylamino)-4-isothiocyanato-azobenzol-2-sulfonsäure chemisch modifiziert. Das so modifizierte Antithrombin wird der Gelchromatograhie an einer Sephadex-G-25-Säule unterworfen. Die Struktur des modifizierten Antithrombins und seine biologische Aktivität werden analysiert. Dabei werden folgende Befunde erhalten:

a) Das chemisch modifizierte Antithrombin besitzt nur noch 25 % seiner Heparin-Cofaktor-Aktivität, aber seine Inhibitor-Aktivität für Thrombin in Abwesenheit von Heparin (progressive Inhibitor-Aktivität) ist praktisch unverändert.

b) Das chemisch modifizierte Antithrombin wird carboxymethyliert und mit Trypsin behandelt. Die so erhaltenen Peptide werden der HPLC unterworfen. Bei einer Wellenlänge von 436 nm werden drei Peptide detektiert. Aus der Strukturanalyse ergibt sich, dass diese drei farbigen Peptide den Aminosäurebausteinen 91 bis 111, 115 bis 129 und 133 bis 139 entsprechen und dass selektiv die Bausteine Lys-107, Lys-125 und Lys-136 durch 4'-(N,N-Dimethylamino)-4-isothiocyanato-azobenzol-2-sulfonsäure modifiziert werden.

c) Wird Antithrombin vor der Einwirkung von 4'-(N,N-Dimethylamino)-4-isothiocyanato-azobenzol-2-sulfonsäure mit Heparin inkubiert, so wird die chemische Modifizierung der Bausteine Lys-107 bzw. Lys-125 bzw. Lys-136 zu 98 % bzw. 88 % bzw. 93 % inhibiert.

d) Die Bausteine Lys-107, Lys-125 und Lys-136 des menschlichen Antithrombins haben demnach für die Bindung des Heparins besondere Bedeutung.

Beispiel 12: In analoger Weise wie in den Beispielen 9 bis 11 beschrieben kann auch eine andere Verbindung der Formel I oder ein Salz einer Verbindung der Formel I, beispielsweise gemäss den

Beispielen 1 bis 8, zur chemischen Modifizierung eines Proteins verwendet werden.

Beispiel 13: Die Untersuchung der Bindungsstelle für Heparin am menschlichen Antithrombin mittels einer Vorrichtung der in Abbildung 1 gezeigten Art kann wie folgt vorgenommen werden:

a) Menschliches Antithrombin (432 Aminosäuren) und der Heparin-Antithrombin-Komplex werden nacheinander jeweils wie unter den Schritten b) bis g) nachfolgend beschrieben behandelt. Die in diesen Schritten benutzte Vorrichtung enthält die schematisch in Abbildung 1 gezeigten Komponenten. Der Reaktor 15 besteht aus Glas. Der Detektor 18 arbeitet auf der Grundlage der Absorption von Licht der Wellenlänge 436 nm. Die Verbindungen 1 und 3 bis 13 bestehen aus Glas. Die Verbindung 2 besteht aus vakuumbeständigem Schlauch. Als Vakuumpumpe 25 wird eine Oelpumpe verwendet. Als Entsalzungseinrichtung 19 dient eine Sephadex-G-25-Säule.

b) Stufe der chemischen Modifizierung

Lösungsmittel: 50 mM Natriumhydrogencarbonatlösung (pH = 8,3);
Probe 1: Antithrombin, 250 μg;
Probe 2: Heparin-Antithrombin-Komplex (2:1 Gewichtsteile), 750 μg (250 μg Antithrombin und 500 μg Heparin);
Reagens der Formel I für die chemische Modifizierung: 1 mM Lösung von 4′-(N,N-Dimethylamino)-4-isothiocyanato-azobenzol-2-sulfonsäure im erwähnten Lösungsmittel;
Reaktionsvolumen: 200 μl;
Reaktionstemperatur: +25°;
Reaktionszeit: 7,5 Minuten.

c) Stufe der Entsal zung

Säule: Sephadex-G-25 (Länge: 3,5 cm; Innendurchmesser: 1 cm);
Laufmittel: 50 mM Ammoniumhydrogencarbonatlösung (pH = 8,0).

d) Stufe der Denaturierung

Lösungsmittel: 0,5 M α,α,α-Tris(hydroxymethyl)methylamin-hydrochloridlösung ("Tris-hydrochlorid"-lösung) (pH = 8,4) und 5 M Guanidin-hydrochlorid-lösung;
Reaktionsvolumen: 200 μl;
Reagens für die Reduktion: threo-1,4-Dimercapto-2,3-butandiol (Dithiothreitol, DTT), 1 mg;
Reduktionstemperatur: +37 °;
Reduktionszeit: 2 Stunden;
Reagens für die Carboxymethylierung: Iodessigsäure, 2 mg;
Carboxymethylierungs-Temperatur: +37°;
Carboxymethylierungs-Zeit: 15 Minuten.

e) Stufe der Entsalzung

Säule und Laufmittel wie bei Stufe c).

f) Stufe der Proteasebehandlung

Protease: Trypsin, 10 μg;
Lösungsmittel: 50 mM Ammoniumhydrogencarbonatlösung (pH = 8,0);
Reaktionsvolumen: 100 μl;
Reaktionstemperatur: +37°;
Reaktionszeit: 2 Stunden.

g) HPLC-Stufe

Säule: Vydac C-18 für Peptide und Proteine;
Säulentemperatur: +25°;
Lösungsmittel A: 17,5 mM Natriumacetat (pH = 5,0);
Lösungsmittel B: Acetonitril;
Gradient: linear von 10 % B bis 70 % B in 30 Minuten;
Fliessgeschwindigkeit: 1 ml pro Minute;
Detektor: Absorption von Licht der Wellenlänge 436 nm.

h) Der Vergleich der Peak-Muster in den beiden Chromatogrammen der Proben 1 bzw. 2 und die Sequenzanalyse der farbigen Peptide, die den Peaks entsprechen, welche lediglich im Chromatogramm der Probe 1 auftreten, zeigen, dass die Aminosäure-Bausteine Lys-107, Lys-125 und Lys-136 im Bereich der Bindungsstelle für Heparin des menschlichen Antithrombins liegen.

Beispiel 14: In analoger Weise wie in Beispiel 13 beschrieben kann die Untersuchung der Bindungsstelle für Hirudin am menschlichen Thrombin mittels der in Beispiel 13 beschriebenen Vorrichtung vorgenommen werden. Menschliches Thrombin (Probe 1; 250 μg) und der Hirudin-Thrombin-Komplex [Probe 2; 750 μg; molares Verhältnis (Hirudin:Thrombin) = (1,2:1)] werden nacheinander wie in Beispiel 13 beschrieben behandelt, wobei jeweils die Schritte b) bis g) in analoger Weise durchgeführt werden. Der

Vergleich der Peak-Muster in den beiden Chromatogrammen der Proben 1 bzw. 2 und die Sequenzanalyse der farbigen Peptide, die den Peaks entsprechen, welche lediglich im Chromatogramm der Probe 1 auftreten, zeigen, dass die Aminosäure-Bausteine Lys-21, Lys-52, Lys-65, Lys-106, Lys-107 und Lys-154 im Bereich der Bindungsstelle für Hirudin des menschlichen Thrombins liegen.

Beispiel 15: In analoger Weise wie in den Beispielen 13 und 14 beschrieben kann auch eine andere Verbindung der Formel I oder ein Salz einer Verbindung der Formel I, beispielsweise gemäss den Beispielen 1 bis 8, als Reagens für die chemische Modifizierung in Stufe b) der in der in Beispiel 13 beschriebenen Vorrichtung durchgeführten Untersuchung verwendet werden.

## Patentansprüche

1. Eine Verbindung der Formel

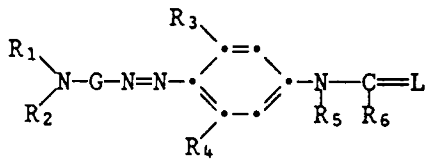

(I),

worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine gegebenenfalls substituierte 1,4-Phenylengruppe oder eine gegebenenfalls substituierte 1,4-Naphthylengruppe steht und worin entweder $R_5$ und $R_6$ gemeinsam für eine zusätzliche Bindung stehen und L ein Sauerstoff- oder ein Schwefelatom bedeutet oder worin $R_5$ Wasserstoff ist, $R_6$ Halogenmethyl bedeutet und L für ein Sauerstoffatom steht, oder ein Salz davon.

2. Eine Verbindung der Formel I gemäss Anspruch 1, worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine gegebenenfalls substituierte 1,4-Phenylengruppe oder eine gegebenenfalls substituierte 1,4-Naphthylengruppe steht, $R_5$ und $R_6$ gemeinsam für eine zusätzliche Bindung stehen und L ein Sauerstoff- oder ein Schwefelatom bedeutet, oder ein Salz davon.

3. Eine Verbindung der Formel I gemäss Anspruch 1, worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine unsubstituierte oder durch Carboxy und/oder Sulfo substituierte 1,4-Phenylengruppe oder eine unsubstituierte oder durch Carboxy und/oder Sulfo substituierte 1,4-Naphthylengruppe steht und worin entweder $R_5$ und $R_6$ gemeinsam für eine zusätzliche Bindung stehen und L ein Sauerstoff- oder ein Schwefelatom bedeutet oder worin $R_5$ Wasserstoff ist, $R_6$ Halogenmethyl bedeutet und L für ein Sauerstoffatom steht, oder ein Salz davon.

4. Eine Verbindung der Formel I gemäss Anspruch 1, worin $R_1$ Niederalkyl darstellt, $R_2$ Niederalkyl ist, $R_3$ Wasserstoff, Carboxy oder Sulfo bedeutet, $R_4$ Carboxy oder Sulfo ist, G für eine unsubstituierte oder durch Carboxy und/oder Sulfo substituierte 1,4-Phenylengruppe oder eine unsubstituierte oder durch Carboxy und/oder Sulfo substituierte 1,4-Naphthylengruppe steht, $R_5$ und $R_6$ gemeinsam für eine zusätzliche Bindung stehen und L ein Sauerstoff- oder ein Schwefelatom bedeutet, oder ein Salz davon.

5. Eine Verbindung der Formel I gemäss Anspruch 1, worin $R_1$ $C_1$-$C_4$-Alkyl darstellt, $R_2$ $C_1$-$C_4$-Alkyl ist, $R_3$ Wasserstoff bedeutet, $R_4$ für Sulfo steht, G eine unsubstituierte 1,4-Phenylengruppe darstellt und worin entweder $R_5$ und $R_6$ gemeinsam für eine zusätzliche Bindung stehen und L ein Schwefelatom bedeutet oder worin $R_5$ Wasserstoff ist, $R_6$ Iodmethyl bedeutet und L für ein Sauerstoffatom steht, oder ein Salz davon.

6. Eine Verbindung der Formel I gemäss Anspruch 1, worin $R_1$ $C_1$-$C_4$-Alkyl darstellt, $R_2$ $C_1$-$C_4$-Alkyl ist, $R_3$ Wasserstoff bedeutet, $R_4$ für Sulfo steht, G eine unsubstituierte 1,4-Phenylengruppe darstellt, $R_5$ und $R_6$ gemeinsam für eine zusätzliche Bindung stehen und L ein Schwefelatom bedeutet, oder ein Salz davon.

**7.** 4′-(N,N-Dimethylamino)-4-isothiocyanato-azobenzol-2-sulfonsäure oder ein Salz davon.

**8.** 4′-(N,N-Dimethylamino)-4-(N-iodacetyl)amino-azobenzol-2-sulfonsäure oder ein Salz davon.

**9.** 4′-(N,N-Dimethylamino)-4-isothiocyanato-azobenzol-2,6-disulfonsäure, 4-(N,N-Dimethylamino)naphthalin-1-azo-(4′-isothiocyanatobenzol-2′-sulfonsäure), 4′-(N,N-Dimethylamino)-4-isocyanato-azobenzol-2-sulfonsäure, 4′-(N,N-Dimethylamino)-4-isocyanato-azobenzol-2,6-disulfonsäure, 4-(N,N-Dimethylamino)-naphthalin-1-azo-(4′-isocyanatobenzol-2′-sulfonsäure) oder jeweils ein Salz davon.

**10.** 4′-(N,N-Dimethylamino)-4-(N-iodacetyl)amino-azobenzol-2,6-disulfonsäure oder 4-(N,N-Dimethylamino)-naphthalin-1-azo-[4′-(N-iodacetyl)aminobenzol-2′-sulfonsäure] oder jeweils ein Salz davon.

**11.** Verfahren zur Herstellung einer Verbindung der Formel I oder eines Salzes davon gemäss einem der Ansprüche 1 bis 10, dadurch gekennzeichnet, dass man in einer Verbindung der Formel

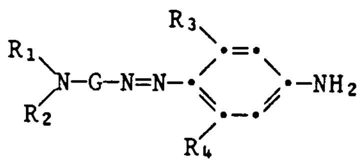 (II)

oder einem Salz davon die $NH_2$-Gruppe in die Gruppe der Formel

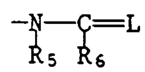 (Ic)

überführt und gewünschtenfalls ein verfahrensgemäss erhältliches Isomerengemisch in die Komponenten auftrennt und das gewünschte Isomere I isoliert, ein verfahrensgemäss erhältliches Enantiomeren- oder Diastereomerengemisch in die Enantiomeren oder Diastereomeren aufspaltet und das gewünschte Enantiomere oder Diastereomere isoliert und/oder eine verfahrensgemäss erhältliche freie Verbindung I in ein Salz überführt oder ein verfahrensgemäss erhältliches Salz in die freie Verbindung I oder in ein anderes Salz umwandelt.

**12.** Eine Verbindung der Formel

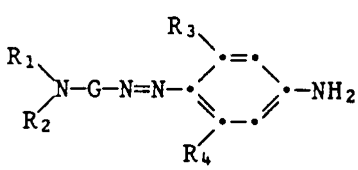 (II),

worin $R_1$ Niederalkyl darstellt, $R_2$ Niederalkyl ist, $R_3$ Wasserstoff, Carboxy oder Sulfo bedeutet, $R_4$ Carboxy oder Sulfo ist und G für eine gegebenenfalls substituierte 1,4-Phenylengruppe oder eine gegebenenfalls substituierte 1,4-Naphthylengruppe steht, oder ein Salz davon.

**13.** Eine Verbindung der Formel II gemäss Anspruch 12, worin $R_1$ Niederalkyl darstellt, $R_2$ Niederalkyl ist, $R_3$ Wasserstoff, Carboxy oder Sulfo bedeutet, $R_4$ Carboxy oder Sulfo ist und G für eine unsubstituierte oder durch Carboxy und/oder Sulfo substituierte 1,4-Phenylengruppe oder eine unsubstituierte oder durch Carboxy und/oder Sulfo substituierte 1,4-Naphthylengruppe steht, oder ein Salz davon.

**14.** Eine Verbindung der Formel II gemäss Anspruch 12, worin $R_1$ $C_1$-$C_4$-Alkyl darstellt, $R_2$ $C_1$-$C_4$-Alkyl ist, $R_3$ Wasserstoff bedeutet, $R_4$ für Sulfo steht und G eine unsubstituierte 1,4-Phenylengruppe darstellt, oder ein Salz davon.

**15.** 4-Amino-4'-(N,N-dimethylamino)-azobenzol-2-sulfonsäure oder ein Salz davon.

**16.** 4-Amino-4'-(N,N-dimethylamino)-azobenzol-2,6-disulfonsäure oder 4-(N,N-Dimethylamino)naphthalin-1-azo-(4'-aminobenzol-2'-sulfonsäure) oder jeweils ein Salz davon.

**17.** Verfahren zur Herstellung einer Verbindung der Formel II oder eines Salzes davon gemäss einem der Ansprüche 12 bis 16, dadurch gekennzeichnet, dass man eine Verbindung der Formel

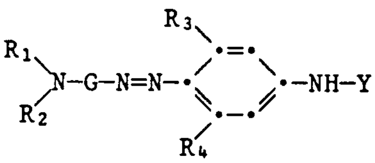 (III),

worin Y für einen Acylrest steht, oder ein Salz davon hydrolysiert und gewünschtenfalls ein verfahrensgemäss erhältliches Isomerengemisch in die Komponenten auftrennt und das gewünschte Isomere II isoliert, ein verfahrensgemäss erhältliches Enantiomeren- oder Diastereomerengemisch in die Enantiomeren oder Diastereomeren aufspaltet und das gewünschte Enantiomere oder Diastereomere isoliert und/oder eine verfahrensgemäss erhältliche freie Verbindung II in ein Salz überführt oder ein verfahrensgemäss erhältliches Salz in die freie Verbindung II oder in ein anderes Salz umwandelt.

**18.** Verfahren für die chemische Modifizierung eines Proteins, dadurch gekennzeichnet, dass man das Protein mit einer Verbindung gemäss einem der Ansprüche 1 bis 10 oder einem Salz davon umsetzt.

**19.** Verfahren für die mit einer Anfärbung verbundene chemische Modifizierung eines Proteins gemäss Anspruch 18, dadurch gekennzeichnet, dass man wie in Anspruch 18 angegeben verfährt.

**20.** Verfahren für die chemische Modifizierung von Lysin-Bausteinen eines Proteins, dadurch gekennzeichnet, dass man das Protein mit 4'-(N,N-Dimethylamino)-4-isothiocyanato-azobenzol-2-sulfonsäure umsetzt.

**21.** Verfahren für die mit einer Anfärbung verbundene chemische Modifizierung von Lysin-Bausteinen eines Proteins gemäss Anspruch 20, dadurch gekennzeichnet, dass man wie in Anspruch 20 angegeben verfährt.

**22.** Verfahren für die chemische Modifizierung von Cystein-Bausteinen eines Proteins, dadurch gekennzeichnet, dass man das Protein mit 4'-(N,N-Dimethylamino)-4-(N-iodacetyl)amino-azobenzol-2-sulfonsäure umsetzt.

**23.** Verfahren für die mit einer Anfärbung verbundene chemische Modifizierung von Cystein-Bausteinen eines Proteins gemäss Anspruch 22, dadurch gekennzeichnet, dass man wie in Anspruch 22 angegeben verfährt.

**24.** Verwendung einer Verbindung gemäss einem der Ansprüche 1 bis 10 oder eines Salzes davon als Hilfsmittel bei der Untersuchung eines Proteins, als Reagens für die chemische Modifizierung eines Proteins, für die mit einer Anfärbung verbundene chemische Modifizierung eines Proteins und/oder als Reagens in dem in Anspruch 31 erwähnten Schritt der chemischen Modifizierung, der in einer Vorrichtung gemäss Anspruch 31 durchgeführt wird.

**25.** Verwendung von 4'-(N,N-Dimethylamino)-4-isothiocyanato-azobenzol-2-sulfonsäure gemäss Anspruch 24.

**26.** Verwendung von 4'-(N,N-Dimethylamino)-4-(N-iodacetyl)amino-azobenzol-2-sulfonsäure gemäss Anspruch 24.

**27.** Verwendung einer Verbindung gemäss einem der Ansprüche 12 bis 16 oder eines Salzes davon als Ausgangsstoff für die Herstellung einer Verbindung der Formel I oder eines Salzes davon.

**28.** Die nach dem Verfahren gemäss einem der Ansprüche 11, 17 und 28 verfahrensgemäss verwendeten neuen Ausgangsstoffe, gebildeten neuen Zwischenprodukte und erhältlichen neuen Endstoffe.

**29.** Eine Vorrichtung zur automatischen Durchführung der Sequenz, bestehend aus der chemischen Modifizierung eines Proteins mittels einer Verbindung I gemäss einem der Ansprüche 1 bis 10 oder eines Salzes davon, aus einer gegebenenfalls vorzunehmenden Denaturierung des chemisch modifizierten Proteins, aus der Proteasebehandlung des chemisch modifizierten und gegebenenfalls denaturierten Proteins und aus der hochauflösenden Flüssigkeitschromatographie (HPLC) des nach der Proteasebehandlung erhaltenen Peptidgemisches, umfassend eine Spritzenpumpe (Einspritzeinrichtung, "syringe pump") (24), welche durch eine Verbindung (1) mit einem Reaktor (15), angeschlossen ist, welcher Reaktor (15) mit einem Schraubdeckel (16) versehen und in einem Heizmantel (14) angeordnet ist, eine Vakuumpumpe (25), die mittels einer Verbindung (2) die Evakuierung des Reaktors (15) zum Zwecke der Konzentration des Reaktorinhalts ermöglicht, eine Verbindung (3), die dazu dient, mittels eines schwachen Inertgasüberdruckes den Inhalt des Reaktors (15), wenn der Teilschritt der chemischen Modifizierung oder der gegebenenfalls auszuführende Teilschritt der Denaturierung ausgeführt worden ist, durch ein Ventil (26), eine Verbindung (5), ein Ventil (27) und eine Verbindung (9) zu einer Entsalzungseinrichtung (19) zu überführen, oder, wenn der Teilschritt der Proteasebehandlung ausgeführt worden ist, durch das Ventil (26), die Verbindung (5), das Ventil (27) und eine Verbindung (7) zu einer HPLC-Komponente (21), an welche eine Zeichenkomponente ("Plotter") (22) und, mittels einer Verbindung (8), ein Fraktionssammler (20) angeschlossen sind, zu überführen, oder die dazu dient, Waschflüssigkeit, die im Reaktor (15) enthalten ist, durch das Ventil (26) und eine Verbindung (11) in einen Abfallflüssigkeitsbehälter (17) zu überführen, eine Pumpe (23), die Laufmittel durch die Verbindung (6), das Ventil (27) und die Verbindung (9) zur Entsalzungseinrichtung (19) fördert, eine Verbindung (10), die dazu dient, das aus der Entsalzungseinrichtung (19) austretende Eluat zu einem Detektor (18) zu leiten, welcher dazu dient, ein Ventil (28) in der Weise einzustellen, dass das aus der Entsalzungseinrichtung (19) austretende Eluat, wenn es das im Schritt der chemischen Modifizierung hergestellte chemisch modifizierte Protein oder das gegebenenfalls im Denaturierungsschritt hergestellte denaturierte chemisch modifizierte Protein enthält, durch eine Verbindung (4) in den Reaktor (15) gelangt und, wenn es kein solches chemisch modifiziertes Protein oder denaturiertes chemisch modifiziertes Protein enthält, durch eine Verbindung (12) in den Abfallflüssigkeitsbehälter (17) gelangt, und eine Verbindung (13), die den Transport des Eluats vom Detektor (18) zum Ventil (28) ermöglicht.

**30.** Verfahren zur automatischen Durchführung der in Anspruch 29 erwähnten Sequenz, dadurch gekennzeichnet, dass das Protein in einer Vorrichtung gemäss Anspruch 29 bearbeitet wird.

**31.** Verfahren gemäss Anspruch 30, dadurch gekennzeichnet, dass man im Schritt der chemischen Modifizierung das Protein 4'-(N,N-Dimethylamino)-4-isothiocyanato-azobenzol-2-sulfonsäure umsetzt.

**32.** Verfahren gemäss Anspruch 30, dadurch gekennzeichnet, dass man im Schritt der chemischen Modifizierung das Protein mit 4'-(N,N-Dimethylamino)-4-(N-iodacetyl)amino-azobenzol-2-sulfonsäure umsetzt.

**33.** Verwendung einer Vorrichtung gemäss Anspruch 29 zur automatischen Durchführung der in Anspruch 29 erwähnten Sequenz.

**34.** Verfahren zur Herstellung einer Vorrichtung gemäss Anspruch 29, dadurch gekennzeichnet, dass die in Anspruch 29 erwähnten Komponenten der Vorrichtung in der in Anspruch 29 beschriebenen Weise kombiniert werden, um die gewünschte Vorrichtung zu ergeben.

## Claims

1. A compound of formula

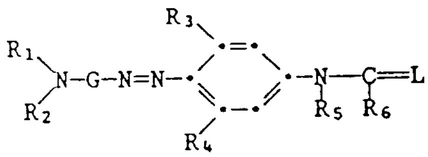

(I),

wherein $R_1$ is lower alkyl, $R_2$ is lower alkyl, $R_3$ is hydrogen, carboxy or sulfo, $R_4$ is carboxy or sulfo, G is an unsubstituted or substituted 1,4-phenylene group or an unsubstituted or substituted 1,4-naphthylene group, and wherein either $R_5$ and $R_6$ together are an additional bond and L is an oxygen or sulfur atom or wherein $R_5$ is hydrogen, $R_6$ is halomethyl and L is an oxygen atom, or a salt thereof.

2. A compound of formula I according to claim 1 wherein $R_1$ is lower alkyl, $R_2$ is lower alkyl, $R_3$ is hydrogen, carboxy or sulfo, $R_4$ is carboxy or sulfo, G is an unsubstituted or substituted 1,4-phenylene group or an unsubstituted or substituted 1,4-naphthylene group, $R_5$ and $R_6$ together are an additional bond and L is an oxygen or sulfur atom, or a salt thereof.

3. A compound of formula I according to claim 1 wherein $R_1$ is lower alkyl, $R_2$ is lower alkyl, $R_3$ is hydrogen, carboxy or sulfo, $R_4$ is carboxy or sulfo, G is an unsubstituted or carboxy- and/or sulfo-substituted 1,4-phenylene group or an unsubstituted or carboxy- and/or sulfo-substituted 1,4-naphthylene group and wherein either $R_5$ and $R_6$ together are an additional bond and L is an oxygen or sulfur atom, or wherein $R_5$ is hydrogen, $R_6$ is halomethyl and L is an oxygen atom, or a salt thereof.

4. A compound of formula I according to claim 1 wherein $R_1$ is lower alkyl, $R_2$ is lower alkyl, $R_3$ is hydrogen, carboxy or sulfo, $R_4$ is carboxy or sulfo, G is an unsubstituted or carboxy- and/or sulfo-substituted 1,4-phenylene group or an unsubstituted or carboxy- and/or sulfo-substituted 1,4-naphthylene group, $R_5$ and $R_6$ together are an additional bond and L is an oxygen or sulfur atom, or a salt thereof.

5. A compound of formula I according to claim 1 wherein $R_1$ is $C_1$-$C_4$alkyl, $R_2$ is $C_1$-$C_4$alkyl, $R_3$ is hydrogen, $R_4$ is sulfo, G is an unsubstituted 1,4-phenylene group and wherein either $R_5$ and $R_6$ together are an additional bond and L is a sulfur atom, or wherein $R_5$ is hydrogen, $R_6$ is iodomethyl and L is an oxygen atom, or a salt thereof.

6. A compound of formula I according to claim 1 wherein $R_1$ is $C_1$-$C_4$alkyl $R_2$ is $C_1$-$C_4$alkyl, $R_3$ is hydrogen, $R_4$ is sulfo, G is an unsubstituted 1,4-phenylene group, $R_5$ and $R_6$ together are an additional bond and L is a sulfur atom, or a salt thereof.

7. 4'-(N,N-Dimethylamino)-4-isothiocyanato-azobenzene-2-sulfonic acid or a salt thereof.

8. 4'-(N,N-Dimethylamino)-4-(N-iodoacetyl)-amino-azobenzene-2-sulfonicacid or a salt thereof.

9. 4'-(N,N-Dimethylamino)-4-isothiocyanato-azobenzene-2,6-disulfonic acid, 4-(N,N-dimethylamino)-naphthalene-1-azo-(4'-isothiocyanatobenzene-2'-sulfonic acid), 4'-(N,N-dimethylamino)-4-isocyanato-azobenzene-2-sulfonic acid, 4'-(N,N-dimethylamino)-4-isocyanato-azobenzene-2,6-disulfonic acid, 4-(N,N-dimethylamino)-naphthalene-1-azo-(4'-isocyanatobenzene-2'-sulfonic acid), or in each case a salt thereof.

10. 4'-(N,N-Dimethylamino)-4-(N-iodoacetyl)-amino-azobenzene-2,6-disulfonic acid or 4-(N,N-dimethylamino)-naphthalene-1-azo-[4'-(N-iodoacetyl)-aminobenzene-2'-sulfonic acid] or in each case a salt thereof.

11. A process for the preparation of a compound of formula I or a salt thereof according to any one of claims 1 to 10, which process comprises: in a compound of formula

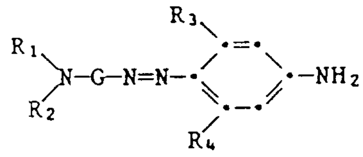

(II)

or in a salt thereof, converting the $NH_2$ group into a group of formula

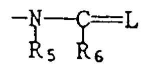

(Ic)

and, if desired, separating a mixture of isomers obtainable in accordance with the process into the components and isolating the desired isomer I, resolving a mixture of enantiomers or diastereoisomers obtainable in accordance with the process into the enantiomers or diastereoisomers and isolating the desired enantiomer or diastereoisomer, and/or converting a free compound I obtainable in accordance with the process into a salt or converting a salt obtainable in accordance with the process into the free compound I or into a different salt.

**12.** A compound of formula

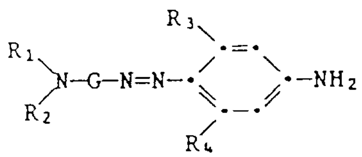

(II)

wherein $R_1$ is lower alkyl, $R_2$ is lower alkyl, $R_3$ is hydrogen, carboxy or sulfo, $R_4$ is carboxy or sulfo, and G is an unsubstituted or substituted 1,4-phenylene group or an unsubstituted or substituted 1,4-naphthylene group, or a salt thereof.

**13.** A compound of formula II according to claim 12 wherein $R_1$ is lower alkyl, $R_2$ is lower alkyl, $R_3$ is hydrogen, carboxy or sulfo, $R_4$ is carboxy or sulfo, and G is an unsubstituted or carboxy- and/or sulfo-substituted 1,4-phenylene group or an unsubstituted or carboxy- and/or sulfo-substituted 1,4-naphthylene group, or a salt thereof.

**14.** A compound of formula II according to claim 12 wherein $R_1$ is $C_1$-$C_4$alkyl, $R_2$ is $C_1$-$C_4$alkyl, $R_3$ is hydrogen, $R_4$ is sulfo, and G is an unsubstituted 1,4-phenylene group, or a salt thereof.

**15.** 4-Amino-4'-(N,N-dimethylamino)-azobenzene-2-sulfonic acid or a salt thereof.

**16.** 4-Amino-4'-(N,N-dimethylamino)-azobenzene-2,6-disulfonic acid or 4-(N,N-dimethylamino)-naphthalene-1-azo-(4'-aminobenzene-2'-sulfonic acid) or in each case a salt thereof.

**17.** A process for the preparation of a compound of formula II or a salt thereof according to any one of claims 12 to 16, which process comprises hydrolysing a compound of formula

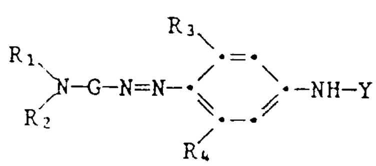

(III),

wherein Y is an acyl radical, or a salt thereof and, if desired, separating a mixture of isomers obtainable

in accordance with the process into the components and isolating the desired isomer II, resolving a mixture of enantiomers or diastereoisomers obtainable in accordance with the process into the enantiomers or diastereoisomers and isolating the desired enantiomer or diastereoisomer, and/or converting a free compound II obtainable in accordance with the process into a salt or converting a salt obtainable in accordance with the process into the free compound II or into a different salt.

**18.** A process for the chemical modification of a protein, wherein the protein is reacted with a compound according to any one of claims 1 to 10 or with a salt thereof.

**19.** A process for the chemical modification of a protein associated with coloration, according to claim 18, wherein the procedure is as indicated in claim 18.

**20.** A process for the chemical modification of lysine building blocks of a protein, wherein the protein is reacted with 4'-(N,N-dimethylamino)-4-isothiocyanato-azobenzene-2-sulfonic acid.

**21.** A process for the chemical modification of lysine building blocks of a protein associated with coloration, according to claim 20, wherein the procedure is as indicated in claim 20.

**22.** A process for the chemical modification of cysteine building blocks of a protein, wherein the protein is reacted with 4'-(N,N-dimethylamino)-4-(N-iodoacetyl)-amino-azobenzene-2-sulfonic acid.

**23.** A process for the chemical modification of cysteine building blocks of a protein associated with coloration, according to claim 22, wherein the procedure is as indicated in claim 22.

**24.** The use of a compound according to any one of claims 1 to 10 or a salt thereof as an adjunct in the investigation of a protein, as a reagent for the chemical modification of a protein, for the chemical modification of a protein associated with coloration, and/or as a reagent in the chemical modification step mentioned in claim 31 that is carried out in a device according to claim 31.

**25.** The use of 4'-(N,N-dimethylamino)-4-isothiocyanato-azobenzene-2-sulfonic acid according to claim 24.

**26.** The use of 4'-(N,N-dimethylamino)-4-(N-iodoacetyl)-amino-azobenzene-2-sulfonic acid according to claim 24.

**27.** The use of a compound according to any one of claims 12 to 16 or a salt thereof as a starting material for the preparation of a compound of formula I or a salt thereof.

**28.** The novel starting materials used in the process, the novel intermediates formed and novel end products obtainable by the process according to any one of claims 11, 17 and 28.

**29.** A device designed to carry out automatically the sequence consisting of the chemical modification of a protein by means of a compound of the formula I according to any one of claims 1 to 10 or a salt thereof, the optional denaturation of the chemically modified protein, the protease treatment of the chemically modified and optionally denatured protein, and the high-performance liquid chromatography (HPLC) of the peptide mixture resulting from the protease treatment step, comprising a syringe pump (24), which component (24) is connected by a connection (1) to a reactor (15), which reactor (15) is equipped with a screw cap (16) and is installed within a heating-jacket (14), a vacuum pump (25), which component (25) enables by means of a connection (2) the evacuation of the reactor (15) for the purpose of the concentration of the contents of the reactor, a connection (3), which component (3), by means of a slight excess pressure of inert gas, serves to deliver the contents of the reactor (15), when the chemical modification step or the optional denaturation step has been carried out, through a valve (26), a connection (5), a valve (27) and a connection (9) to a desalting unit (19), or serves to deliver the contents of the reactor (15), when the protease treatment step has been carried out, through the valve (26), the connection (5), the valve (27) and a connection (7) to a HPLC component (21), to which component (21) a plotter (22) and, by means of a connection (8), a fraction collector (20) are attached, or serves to deliver washing fluid contained in the reactor (15) through the valve (26) and a connection (11) to a waste fluid reservoir (17), a pump (23), which component (23) delivers eluant through the connection (6), the valve (27) and the connection (9) to the desalting unit (19), a connection (10), which

component (10) serves to deliver the eluate which is eluted from the desalting unit (19) to a detector (18), which component (18) serves to control a valve (28) in such a way that the eluate which is eluted from the desalting unit (19), when it contains the chemically modified protein prepared in the chemical modification step or the denatured chemically modified protein optionally prepared in the denaturation step, is delivered through a connection (4) into the reactor (15), and when it contains no such chemically modified protein or denatured chemically modified protein is delivered through a connection (12) into the waste fluid reservoir (17), and a connection (13), which component (13) serves to deliver the eluate from the detector (18) to the valve (28).

**30.** A process for carrying out automatically the sequence mentioned in claim 29, wherein the protein is processed in a device according to claim 29.

**31.** A process according to claim 30, wherein in the chemical modification step the protein is reacted with 4'-(N,N-dimethylamino)-4-isothiocyanato-azobenzene-2-sulfonic acid.

**32.** A process according to claim 30, wherein in the chemical modification step the protein is reacted with 4'-(N,N-dimethylamino)-4-(N-iodoacetyl)-amino-azobenzene-2-sulfonicacid.

**33.** The use of a device according to claim 29 for carrying out automatically the sequence mentioned in claim 29.

**34.** A process for the manufacture of a device according to claim 29, wherein the components of the device mentioned in claim 29 are combined in the manner described in claim 29 to produce the desired device.

## Revendications

**1.** Composé de formule I :

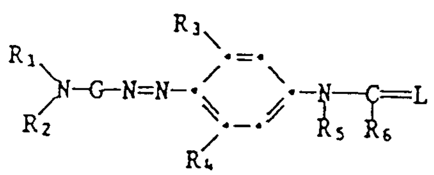

(I),

dans laquelle $R_1$ est un radical alkyle inférieur, $R_2$ est un radical alkyle inférieur, $R_3$ est un hydrogène ou un radical carboxy ou sulfo, $R_4$ est un radical carboxy ou sulfo, G est un groupe 1,4-phénylène éventuellement substitué ou un groupe 1,4-naphtylène éventuellement substitué, et où $R_5$ et $R_6$ représentent en commun une liaison supplémentaire, et L est un atome d'oxygène ou de soufre, ou dans laquelle $R_5$ est un hydrogène, $R_6$ est un radical halogénométhyle, et L est un atome d'oxygène, ou l'un de ses sels.

**2.** Composé de formule I selon la revendication 1, dans lequel $R_1$ est un radical alkyle inférieur, $R_2$ est un radical alkyle inférieur, $R_3$ est un hydrogène, un groupe carboxy ou sulfo, $R_4$ est un groupe carboxy ou sulfo, G est un groupe 1,4-phénylène éventuellement substitué ou un groupe 1,4-naphtylène éventuellement substitué, $R_5$ et $R_6$ forment ensemble une liaison supplémentaire, et L est un atome d'oxygène ou un atome de soufre, ou l'un de ses sels.

**3.** Composé de formule I selon la revendication 1, dans lequel $R_1$ est un radical alkyle inférieur, $R_2$ est un radical alkyle inférieur, $R_3$ est un hydrogène ou un groupe carboxy ou sulfo, $R_4$ est un groupe carboxy ou sulfo, G est un groupe 1,4-phénylène non substitué ou à substitution carboxy et/ou sulfo ou un groupe 1,4-naphtylène non substitué ou à substitution carboxy et/ou sulfo, et où ou bien $R_5$ et $R_6$ forment ensemble une liaison supplémentaire et L est un atome d'oxygène ou un atome de soufre, ou bien $R_5$ est un hydrogène, $R_6$ est un radical halogénométhyle et L est un atome d'oxygène, ou l'un de ses sels.

**4.** Composé de formule I selon la revendication 1, dans lequel $R_1$ est un radical alkyle inférieur, $R_2$ est un radical alkyle inférieur, $R_3$ est un hydrogène, un groupe carboxy ou sulfo, $R_4$ est un groupe carboxy ou sulfo, G est un groupe 1,4-phénylène non substitué ou à substitution carboxy et/ou sulfo ou un groupe 1,4-naphtylène non substitué ou à substitution carboxy et/ou sulfo, $R_5$ et $R_6$ forment ensemble une liaison supplémentaire, et L est un atome d'oxygène ou un atome de soufre, ou l'un de ses sels.

**5.** Composé de formule I selon la revendication 1, dans lequel $R_1$ est un radical alkyle en $C_1$ à $C_4$, $R_2$ est un radical alkyle en $C_1$ à $C_4$, $R_3$ est un hydrogène, $R_4$ est un groupe sulfo, G est un groupe 1,4-phénylène non substitué et où ou bien $R_5$ et $R_6$ forment ensemble une liaison supplémentaire et L est un atome de soufre, ou bien $R_5$ est un hydrogène, $R_6$ est le radical iodométhyle et L est un atome d'oxygène, ou l'un de ses sels.

**6.** Composé de formule I selon la revendication 1, dans lequel $R_1$ est un radical alkyle en $C_1$ à $C_4$, $R_2$ est un radical alkyle en $C_1$ à $C_4$, $R_3$ est un hydrogène, $R_4$ est un groupe sulfo, G est un groupe 1,4-phénylène non substitué et $R_5$ et $R_6$ forment ensemble une liaison supplémentaire et L est un atome de soufre, ou l'un de ses sels.

**7.** Acide 4'-(N,N-diméthylamino)-4-isothiocyanato-azobenzène-2-sulfonique ou l'un de ses sels.

**8.** Acide 4'-(N,N-diméthylamino)-4-(N-iodacétyl)-aminoazobenzène-2-sulfonique ou l'un de ses sels.

**9.** Acide 4'-(N,N-diméthylamino)-4-isothiocyanato-azobenzène-2,6-disulfonique, acide 4-(N,N-diméthylamino)naphtalène-1-azo-(4'-isothiocyanatobenzène-2'-sulfonique), acide 4'-(N,N-diméthylamino)-4-isocyanato-azobenzène-2-sulfonique, acide 4'-(N,N-diméthylamino)-4-isocyanato-azobenzène-2,6-disulfonique, acide 4-(N,N-diméthylamino)naphtalène-1-azo-(4'-isocyanato-benzène-2'-sulfonique), ou un de leurs sels.

**10.** Acide 4'-(N,N-diméthylamino)-4-(N-iodacétyl)-aminoazobenzène-2,6- disulfonique ou acide 4 (N,N-diméthylamino)naphtalène-1-azo[4'-(N-iodacétyl)-aminobenzène-2'-sulfonique], ou un de leurs sels.

**11.** Procédé pour préparer un composé de formule I ou l'un de ses sels selon l'une des revendications 1 à 10, caractérisé en ce que, dans un composé de formule :

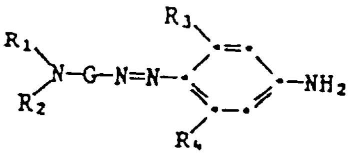

(II)

ou un de ses sels, on convertit le groupe $NH_2$ en le groupe de formule :

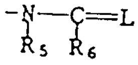

(Ic)

et, si on le souhaite, on sépare en ses constituants un mélange d'isomères pouvant être obtenu par le procédé selon l'invention et on isole l'isomère recherché I, on dédouble en ses énantiomères ou diastéréoisomères un mélange d'énantiomères ou de diastéréoisomères pouvant être obtenu par le procédé selon l'invention et on isole l'énantiomère ou le diastéréoisomère souhaité, et/ou on convertit en un sel un composé libre I pouvant être obtenu par le procédé selon l'invention, ou encore on convertit en le composé libre I ou en un autre sel, un sel pouvant être obtenu par le procédé selon l'invention.

**12.** Composé de formule :

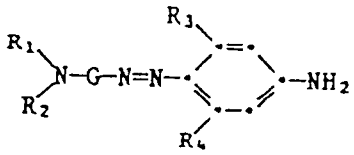

(II)

dans laquelle $R_1$ est un radical alkyle inférieur, $R_2$ est un radical alkyle inférieur, $R_3$ est un hydrogène, un groupe carboxy ou sulfo, $R_4$ est un groupe carboxy ou sulfo, G est un groupe 1,4-phénylène éventuellement substitué ou un groupe 1,4-naphtylène éventuellement substitué, ou l'un de ses sels.

**13.** Composé de formule II selon la revendication 12, dans laquelle $R_1$ est un radical alkyle inférieur, $R_2$ est un radical alkyle inférieur, $R_3$ est un hydrogène, un groupe carboxy ou sulfo, $R_4$ est un groupe carboxy ou sulfo et G est un groupe 1,4-phénylène non substitué ou à substitution carboxy et/ou sulfo ou encore un groupe 1,4-naphtylène non substitué ou à substitution carboxy et/ou sulfo, ou l'un de ses sels.

**14.** Composé de formule II selon la revendication 12, dans lequel $R_1$ est un radical alkyle en $C_1$ à $C_4$, $R_2$ est un radical alkyle en $C_1$ à $C_4$, $R_3$ est un hydrogène, $R_4$ est le groupe sulfo, et G est un groupe 1,4-phénylène non substitué, ou l'un de ses sels.

**15.** Acide 4-amino-4'-(N,N-diméthylamino)-azobenzène-2-sulfonique ou l'un de ses sels.

**16.** Acide 4-amino-4'-(N,N-diméthylamino)-azobenzène-2,6-disulfonique ou acide 4-(N,N-diméthylamino)-naphtalène-1-azo-(4'-aminobenzène-2'-sulfonique) ou l'un de leurs sels.

**17.** Procédé pour préparer un composé de formule II ou un de ses sels, selon l'une des revendications 12 à 16, caractérisé en ce qu'on hydrolyse un composé de formule :

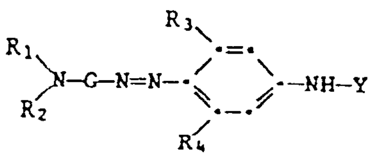

(III),

dans laquelle Y est un radical acyle, ou l'un de ses sels, et, si on le souhaite, on sépare en ses constituants un mélange d'isomères pouvant être obtenu par le procédé selon l'invention, et on isole l'isomère recherché II, on dédouble en les énantiomères ou diastéréoisomères un mélange d'énantiomères ou de diastéréoisomères pouvant être obtenu par le procédé selon l'invention, et on isole l'énantiomère ou le diastéréoisomère souhaité, et/ou on convertit en un sel un composé libre II pouvant être obtenu par le procédé selon l'invention, ou encore on convertit un sel pouvant être obtenu par le procédé selon l'invention en le composé libre II ou en un autre sel.

**18.** Procédé de modification chimique d'une protéine, caractérisé en ce qu'on fait réagir la protéine avec un composé selon l'un des revendications 1 à 10 ou l'un de ses sels.

**19.** Procédé de modification chimique, liée à une coloration, d'une protéine, selon la revendication 18, caractérisé en ce qu'on procède comme indiqué dans la revendication 18.

**20.** Procédé de modification chimique de composants lysine d'une protéine, caractérisé en ce qu'on fait réagir la protéine avec l'acide 4'-(N,N-diméthylamino)-4-isothiocyanato-azobenzène-2-sulfonique.

**21.** Procédé de modification chimique, liée à une coloration de composants lysine d'une protéine, selon la revendication 20, caractérisé en ce qu'on procède comme indiqué dans la revendication 20.

**22.** Procédé de modification chimique de composants cystéine d'une protéine, caractérisé en ce qu'on fait réagir la protéine avec l'acide 4'-(N,N-diméthylamino)-4-(iodacétyl)-aminoazobenzène-2-sulfonique.

**23.** Procédé de modification chimique, liée à une coloration, de composants cystéine d'une protéine, selon la revendication 22, caractérisé en ce qu'on procède comme indiqué dans la revendication 22.

**24.** Utilisation d'un composé selon l'une des revendications 1 à 10 ou d'un de ses sels comme agent auxiliaire lors de l'étude d'une protéine, comme réactif pour la modification chimique d'une protéine, pour la modification chimique, liée à une coloration d'une protéine, et/ou comme réactif dans l'étape mentionnée dans la revendication 31 de modification chimique, qui est mise en oeuvre dans un dispositif selon la revendication 31.

**25.** Utilisation de l'acide 4'-(N,N-diméthylamino)-4-isothiocyanato-azobenzène-2-sulfonique selon la revendication 24.

**26.** Utilisation de l'acide 4'-(N,N-diméthylamino)-4-(iodacétyl)-aminoazobenzène-2-sulfonique selon la revendication 24.

**27.** Utilisation d'un composé selon l'une des revendications 12 à 16 ou d'un de ses sels comme matière de départ pour la préparation d'un composé de formule I ou d'un de ses sels.

**28.** Nouvelles matières de départ, utilisées selon le procédé de l'invention, par le procédé selon l'une des revendications 11, 17 et 28, nouveaux produits intermédiaires formés et nouveaux produits finis pouvant être obtenus.

**29.** Dispositif destiné à la mise en oeuvre automatique de la séquence comprenant la modification chimique d'une protéine à l'aide d'un composé I selon l'une des revendications 1 à 10 ou d'un de ses sels, une dénaturation, qu'il convient éventuellement de mettre en oeuvre, de la protéine, ayant subi une modification chimique, le traitement par une protéase de la protéine ayant subi une modification chimique et éventuellement dénaturée, et la chromatographie en phase liquide hautes performances (CLHP) du mélange de peptides obtenu après le traitement par une protéase, comprenant une pompe à seringue (dispositif d'injection, "syringe pump") (24), qui est connectée par une ligne (1) à un réacteur (15), lequel réacteur (15) est pourvu d'un couvercle fileté (16) et est disposé dans une chemise chauffante (14) ; une pompe à vide (25), qui à l'aide d'une ligne (2) permet de faire le vide dans le réacteur (15) pour assurer la con-centration du contenu du réacteur ; une ligne (3), qui, à l'aide d'une légère surpression d'un gaz inerte, et quand on a exécuté l'étape partielle de modification chimique ou l'étape partielle de dénaturation, qu'il convient eventuellement de mettre en oeuvre, sert à envoyer le contenu du réacteur (15), par une vanne (26), une ligne (5), une vanne (27) et une ligne (9), à une unité d'élimination des sels (19), ou bien, quand on a exécuté l'étape partielle de traitement par une protéase, sert à l'envoyer, par la vanne (26), la ligne (5), la vanne (27) et une ligne (7), à une unité de CLHP (21), à laquelle sont raccordés une table traçante ("plotter") (22), et, par une ligne (8), un collecteur de fractions (20), ou encore qui sert à envoyer un liquide de lavage contenu dans le réacteur (15), par la vanne (26) et une ligne (11), dans un récipient de liquide résiduaire (17); une pompe (23), qui refoule l'éluant par la ligne (6), la vanne (27) et la ligne (9) jusqu'à l'unité d'élimination des sels (19) ; une ligne (10), qui sert à envoyer l'éluat sortant de l'unité d'élimination des sels (19) à un détecteur (18), lequel sert à ajuster une vanne (28) de telle sorte que l'éluat sortant de l'unité d'élimination des sels (19), quand il contient la protéine ayant subi une modification chimique, préparée dans l'étape de modification chimique, ou encore la protéine ayant subi une modification chimique, éventuellement dénaturée dans l'étape de dénaturation, passe par une ligne (4) pour arriver dans le réacteur (15) et, quand il ne contient pas une telle protéine ayant subi une modification chimique ou une telle protéine dénaturée ayant subi une modification chimique, passe par une ligne (12) pour arriver dans le récipient de liquide résiduaire (17) ; et une ligne (13), qui permet le transport de l'éluat du détecteur (18) à la vanne (28).

**30.** Procédé pour la mise en oeuvre automatique de la séquence mentionnée dans la revendication 29, caractérisé en ce que la protéine est traitée dans un dispositif selon la revendication 29.

**31.** Procédé selon la revendication 30, caractérisé en ce que, dans l'étape de modification chimique, on fait réagir la protéine avec l'acide 4'-(N,N-diméthylamino)-4-isothiocyanato-azobenzène-2-sulfonique.

**32.** Procédé selon la revendication 30, caractérisé en ce que, dans l'étape de modification chimique, on fait réagir la protéine avec l'acide 4'-(N,N-dimèthylamino)-4-(N-iodacétyl)-aminoazobenzène-2-sulfonique.

**33.** Utilisation d'un dispositif selon la revendication 29 pour la mise en oeuvre de la séquence mentionnée dans la revendication 29.

**34.** Procédé pour fabriquer un dispositif selon la revendication 29, caractérisé en ce que, pour obtenir le dispositif souhaité, on combine de la manière décrite dans la revendication 29, les composants du dispositif mentionnés dans la revendication 29.

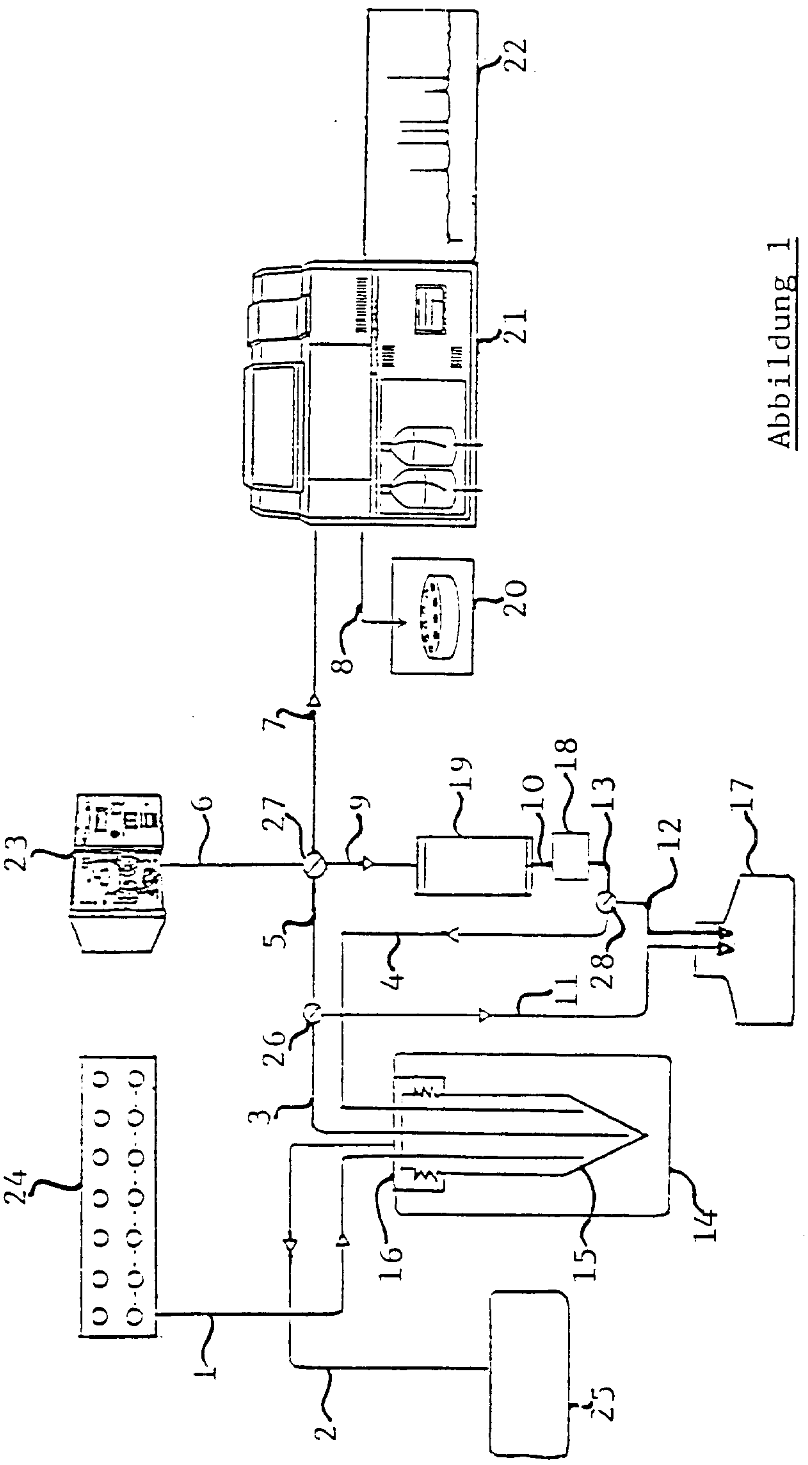


Abbildung 1